# EUROPEAN PATENT SPECIFICATION

(11) **EP 3 849 310 B1**
(45) Date of publication and mention of the grant of the patent: **13.05.2026**
(21) Application number: 19859767.6
(22) Date of filing: 12.09.2019
(51) Int. Cl.: A61K 31/38, A01N 43/18, A61P 35/00, A61K 31/439, A61K 31/519, A61K 45/06, A61K 31/382

(54) **COMBINATION CANCER THERAPIES BASED ON THE MORTALIN TARGETING COMPOUND SHETA2 AND P53 REACTIVATORS OR CDK4/6 INHIBITORS**
KOMBINATIONSKREBSTHERAPIEN, BASIEREND AUF DER MORTALIN-TARGETING-VERBINDUNG SHETA2 UND P53 REAKTIVATOREN ODER CDK4/6 INHIBITOREN
THÉRAPIES DE CANCER DE COMBINAISON BASÉES SUR LE COMPOSE CIBLANT LA MORTALIN SHETA2 ET LES REACTIVATRICES DE P53 OU LES INHIBITEURS DE CDK4/6

(30) Priority: 12.09.2018 US 201862730345 P
(43) Date of publication of application: 21.07.2021
(73) Proprietor: The Board of Regents of the University of Oklahoma, Norman, OK 73019 (US)
(72) Inventor: BENBROOK, Doris, Mangiaracina, Oklahoma City, OK 73165 (US); RAMRAJ, Satish, Kumar, Oklahoma City, OK 73105 (US)
(74) Representative: J A Kemp LLP
(86) International application number: PCT/US2019/050775
(87) International publication number: WO 2020/056109

(56) References cited:
- WO-A1-2017/037576
- WO-A1-2017/159877
- US-B2- 7 612 107
- CHUN KYUNG-HEE ET AL: "The Synthetic Heteroarotinoid SHetA2 Induces Apoptosis in Squamous Carcinoma Cells through a Receptor-independent and Mitochondria-dependent Pathway 1", CANCER RESEARCH, 1 July 2003 (2003-07-01), pages 3826 - 3832, XP055920481, Retrieved from the Internet <URL:https://aacrjournals.org/cancerres/article-pdf/63/13/3826/2504919/ch1303003826.pdf> [retrieved on 20220512]
- RAMRAJ SATISH KUMAR: "Abstract LB-244: Prevention of ovarian cancer tumor establishment with mortalin targeting and p53 reactivator drugs | Cancer Research | American Association for Cancer Research", 1 January 2019 (2019-01-01), XP055920373, Retrieved from the Internet <URL:https://aacrjournals.org/cancerres/article/79/13_Supplement/LB-244/638067/Abstract-LB-244-Prevention-of-ovarian-cancer-tumor> [retrieved on 20220511]
- ROBINSON TYLER J. W. ET AL: "RB1 Status in Triple Negative Breast Cancer Cells Dictates Response to Radiation Treatment and Selective Therapeutic Drugs", vol. 8, no. 11, 1 January 2013 (2013-01-01), pages e78641, XP055870642, Retrieved from the Internet <URL:https://www.ncbi.nlm.nih.gov/pmc/articles/PMC3827056/pdf/pone.0078641.pdf> DOI: 10.1371/journal.pone.0078641
- LI ZONGJUAN ET AL: "Synergistic Antitumor Effect of BKM120 with Prima-1Met Via Inhibiting PI3K/AKT/mTOR and CPSF4/hTERT Signaling and Reactivating Mutant P53", vol. 45, no. 5, 1 January 2018 (2018-01-01), CH, pages 1772 - 1786, XP055920828, ISSN: 1015-8987, Retrieved from the Internet <URL:https://www.karger.com/Article/Pdf/487786> DOI: 10.1159/000487786
- PHILLIPSE WAYNE: "APR-246 potently inhibits tumour growth and overcomes chemoresistance in preclinical models of oesophageal adenocarcinoma | Gut", 1 January 2015 (2015-01-01), XP055920820, Retrieved from the Internet <URL:https://gut.bmj.com/content/64/10/1506.long> [retrieved on 20220512]
- ALI DINA ET AL: "APR-246 exhibits anti-leukemic activity and synergism with conventional chemotherapeutic drugs in acute myeloid leukemia cells : APR-246 in acute myeloid leukemia", EUROPEAN JOURNAL OF HAEMATOLOGY, vol. 86, no. 3, 11 January 2011 (2011-01-11), DK, pages 206 - 215, XP055802488, ISSN: 0902-4441, DOI: 10.1111/j.1600-0609.2010.01557.x
- RAMRAJ ET AL.: "SHetA2 targets mortalin-p53 interaction in differential induction of apoptosis in ovarian cancer over normal cells [abstract] , Proceedings of the American Association for Cancer Research Annual Meeting 2017", CANCER RES 2017, vol. 77, no. 13, April 2017 (2017-04-01), Washington, DC . Philadelphia (PA ): AACR, pages 3225 - 5, XP009527137, DOI: 10.1158/1538-7445.AM2017-3225

## Description

### CROSS-REFERENCE TO RELATED APPLICATIONS

The present application claims priority under 35 U.S.C. § 119(e) to U.S. Serial No. 62/730,345, filed September 12, 2018.

### GOVERNMENT SUPPORT

This invention was made with government support under U.S. National Cancer Institute grants R01 CA196200 and R01 CA200126. The government has certain rights in the invention.

### BACKGROUND

Despite being cancer free after primary cytoreductive surgery and platinum-based chemotherapy, approximately 70% of ovarian cancer patients will relapse after 3 years. Maintenance therapy using the FDA-approved PARP-1 inhibitor, olaparib, in BRCA mutation positive patients, or the angiogenesis inhibitor, bevacizumab, in all other patients, has been shown to prolong time to recurrence, however no improvement in overall survival was found in long term follow-up for bevacizumab or interim analysis of 21% data for olaparib. Both of these therapies have significant side effects that limit dosing and duration of treatment. Furthermore, olaparib caused an increased risk of developing secondary cancers.

A rational approach for developing non-toxic maintenance therapy for ovarian cancer is to restore normal p53 activity. Mutations in the TP53 gene occur with a 96 to 100% frequency in high-grade serous ovarian cancer (HGSOC), the most common and lethal type of ovarian cancer, which can arise from fallopian tube fimbriae. Mutation or loss of p53 protein can reduce cancer therapy response. Currently p53 restoration is a target of multiple anti-cancer drug strategies, including by way of reactivation of the wild type p53 functions in missense mutant p53 (m-p53) and release of p53 from inhibitory proteins. PRIMA-1 ("p53 re-activation and induction of massive apoptosis-1") is a p53 reactivator that modifies amino acids in the m-p53 core domain to restore proper protein conformation and function. PRIMA-1^{MET} (APR-246), a methylated analog of PRIMA-1, exhibited greater cancer cell line cytotoxicity and synergy with chemotherapy, and is undergoing clinical investigation. A first-in-human clinical trial found PRIMA-1^{MET} to have a favorable pharmacokinetic profile, to induce wild type p53 molecular and cellular activities in tumors and to be safe at plasma levels predicted to have therapeutic effects. Currently, clinicaltrials.gov reports multiple ongoing trials evaluating PRIMA-1^{MET} in combination with chemotherapy for several malignancies, including recurrent ovarian cancer.

Another drug in development that can induce p53 activity is SHetA2 (NSC 726189), an orally bioavailable, small molecule drug that disrupts mortalin/p53 complexes in ovarian cancer cells. This drug is currently being developed for clinical trials based on its induction of apoptosis in cancer cells, while its effect on healthy cells is limited to G1 cell cycle arrest. SHetA2 also exhibited chemoprevention activity. Extensive preclinical studies conducted by the US National Cancer Institute (NCI) RAID and RAPID Programs, and others, demonstrated that SHetA2 did not cause mutagenicity, teratogenicity or toxicity, and exhibited a pharmacologic profile suitable for an oral chemoprevention agent. Peer-reviewed NCIR01 and PREVENT grants are supporting first-in-human clinical trials of SHetA2 capsules. Chun KH et al, Cancer Res. 2003 Jul 1;63(13):3826-32 teaches that SHetA2 induces apoptosis in cancer cells and that several retinoid receptor antagonists failed to prevent apoptosis induction by SHetA2.

### SUMMARY OF INVENTION

The present invention is set out in the appended set of claims.

### BRIEF DESCRIPTION OF THE DRAWINGS

Several embodiments of the present disclosure are hereby illustrated in the appended drawings. It is to be noted however, that the appended drawings only illustrate several typical embodiments and are therefore not intended to be considered limiting of the scope of the inventive concepts disclosed herein. The patent or application file contains at least one drawing executed in color. Copies of this patent or patent application publication with color drawing(s) will be provided by the Office upon request and payment of the necessary fee.
FIG. 1. Upregulation of mortalin expression in ovarian cancer. Representative images of mortalin immunohistochemical staining from the GOG Tissue Microarray (TMA) of ovarian cancer tumor progression.
FIG. 2. Upregulation of mortalin expression in ovarian cancer epithelial cells. Comparison of average mortalin immunohistochemical positivity stain scores for epithelial cells in benign, borderline and serous tumor tissues in the GOG Tissue Microarray (TMA) of ovarian cancer tumor progression. *p ≤ 0.05, ***p ≤ 0.001.
FIG. 3. Upregulation of mortalin expression in ovarian cancer. Comparison of average mortalin immunohistochemical positivity stain scores for stromal cells in benign, borderline and serous tumor tissues in the GOG Tissue Microarray (TMA) of ovarian cancer tumor progression. *p ≤ 0.05, ***p ≤ 0.001.
FIG. 4. SHetA2 causes p53 accumulation in the nucleus of cancer cells, but not healthy cells. Immunofluorescence assay was performed on A2780 and OV90 ovarian cancer cell lines and FTSECs (normal fallopian tube secretory epithelial cells) by detecting p53 with Alexa fluor 647 (Red) fluorochrome-conjugated antibody and nuclei were stained with DAPI (blue). Imaging was performed with confocal microscope at 63X.
FIG. 5A. SHetA2 causes p53 accumulation in the nucleus of cancer cells. Immunoblots of p53 in cytoplasmic and nuclear protein fractions A2780 ovarian cancer cells. GAPDH and Histone H3 served as loading controls for cytoplasmic and nuclear fractions, respectively.
FIG. 5B. SHetA2 causes p53 accumulation in the nucleus of cancer cells. Bar graphs of quantified p53 band densities, which were normalized to the loading controls of the western blots in FIG 5A.
FIG. 6A. SHetA2 causes p53 accumulation in the nucleus of cancer cells. Immunoblots of p53 in cytoplasmic and nuclear protein fractions OV90 ovarian cancer cells. GAPDH and Histone H3 served as loading controls for cytoplasmic and nuclear fractions, respectively.
FIG. 6B. SHetA2 causes p53 accumulation in the nucleus of cancer cells. Bar graphs of quantified p53 band densities, which were normalized to the loading controls of the western blots in FIG. 6A.
FIG. 7A. Mitochondrial accumulation of p53 after SHetA2 treatment in cancer cells. p53 levels were examined in western blots of cytoplasmic and mitochondrial fractions of A2780 ovarian cancer cells, which have wild type p53, collected after SHetA2 treatment for 24 h. β-actin and Tom20 served as protein loading controls in cytoplasmic and mitochondrial fractions, respectively.
FIG. 7B. Mitochondrial accumulation of p53 after SHetA2 treatment in cancer cells. Bar graphs of quantified p53 band densities, which were normalized the loading controls of the western blots in FIG. 7A.
FIG. 8A. Mitochondrial accumulation of p53 after SHetA2 treatment in cancer cells. p53 levels were examined in western blots of cytoplasmic and mitochondrial fractions of OVCAR4 ovarian cancer cells, which have missense mutant p53, collected after SHetA2 treatment for 24 h. β-actin and Tom20 served as protein loading controls in cytoplasmic and mitochondrial fractions, respectively.
FIG. 8B. Mitochondrial accumulation of p53 after SHetA2 treatment in cancer cells. Bar graphs of quantified p53 band densities, which were normalized to the loading controls of the western blots in FIG. 8A.
FIG 9A. No nuclear accumulation of p53 after SHetA2 treatment in FTSECs (non-cancerous human fallopian tube secretory epithelial cells). p53 levels were examined in western blots of cytoplasmic and mitochondrial fractions of FTSECs collected after SHetA2 treatment for 24 h. β-actin and Histone H3 served as protein loading controls in cytoplasmic and nuclear fractions, respectively.
FIG. 9B. No nuclear accumulation of p53 after SHetA2 treatment in cancer cells. Bar graphs of quantified p53 band densities, which were normalized to the loading controls in the western blots of FIG. 9A.
FIG. 10A. No mitochondrial accumulation of p53 after SHetA2 treatment in FTSECs (non-cancerous human fallopian tube secretory epithelial cells). p53 levels were examined in western blots of cytoplasmic and mitochondrial fractions of FTSECs collected after SHetA2 treatment for 24 h. β-actin and Tom20 served as protein loading controls in cytoplasmic and mitochondrial fractions, respectively.
FIG. 10B. Mitochondrial accumulation of p53 after SHetA2 treatment in cancer cells. Bar graphs of quantified p53 band densities, which were normalized to β-actin for the cytoplasmic extracts and to Tom20 for the mitochondrial extracts in the western blots of FIG. 9A.
FIG. 11. Reduction of p53 expression reduces sensitivity of cancer cells to SHetA2. Comparison of SHetA2 sensitivity in A2780 ovarian cancer cells transfected with p53 shRNA or empty vector using an MTT assay. Insert is a western blot confirming p53 reduction.
FIG. 12. Overexpression of mortalin reduces sensitivity of cancer cells to SHetA2. Sensitivity of A2780 cells transfected with a mortalin expression plasmid or empty vector were compared using a MTT assay. Insert is a western blot confirming mortalin overexpression.
FIG. 13. Ovarian cancer cells with wild type p53 are more sensitive to SHetA2 in comparison to cells that are p53 null or that express missense p53 mutants. SHetA2 sensitivities of hFTSECs and ovarian cancer cell lines with various p53 status were compared using a MTT cytotoxicity assay.
FIG. 14. Expression of missense p53 mutants increases sensitivity of SKOV3 ovarian cancer cells to PRIMA-1^{MET}. MTT assay to compare PRIMA-1 cytotoxicity in SKOV3 parental cell line and SKOV3 sublines stably transfected with p53 mutant (R248W and R273H).
FIG. 15. PRIMA-1^{MET} reactivates R282W mutant p53 in ovarian cancer cells. MESOV ovarian cancer cells transfected with PG13-Luc plasmid were evaluated with a luciferase reporter assay to measure the effects of SHetA2 and PRIMA-1^{MET} on p53 transcriptional activity. ANOVA: **p ≤ 0.01.
FIG. 16. Isobologram of SHetA2 and PRIMA-1 combination in A2780 ovarian cancer cells (wild type -53). Fold effects of a 2-fold dilution series of a drug combination mixed at 1:1 ratio of the EC₅₀s of each drug were compared with single drug dilution series using the Chou-Talaly method.
FIG. 17. Isobologram of SHetA2 and PRIMA-1 combination in MESOV ovarian cancer cells (R282W mutant p53). Fold effects of a 2-fold dilution series of a drug combination mixed at 1:1 ratio of the EC₅₀s of each drug were compared with single drug dilution series using the Chou-Talaly method.
FIG. 18. Isobologram of SHetA2 and PRIMA-1 combination in SKOV3 R248W ovarian cancer cells (R248W mutant p53). Fold effects of a 2-fold dilution series of a drug combination mixed at 1:1 ratio of the EC₅₀s of each drug were compared with single drug dilution series using the Chou-Talaly method.
FIG. 19. Isobologram of SHetA2 and PRIMA-1 combination in Caov3 ovarian cancer cells (p53 null). Fold effects of a 2-fold dilution series of a drug combination mixed at 1:1 ratio of the EC₅₀s of each drug were compared with single drug dilution series using the Chou-Talaly method.
FIG. 20. Isobologram of SHetA2 and PRIMA-1 combination in non-cancerous human fallopian tube secretory epithelial cells (FTSECs). Fold effects of a 2-fold dilution series of a drug combination mixed at 1:1 ratio of the EC₅₀s of each drug were compared with single drug dilution series using the Chou-Talaly method.
FIG. 21. Isobologram of SHetA2 and PRIMA-1^{MET} combination in MESOV ovarian cancer cells (R282W mutant p53). Fold effects of a 2-fold dilution series of a drug combination mixed at 1:1 ratio of the EC₅₀s of each drug were compared with single drug dilution series using the Chou-Talaly method.
FIG. 22. Isobologram of SHetA2 and PRIMA-1^{MET} combination in non-cancerous human fallopian tube secretory epithelial cells (FTSECs). Fold effects of a 2-fold dilution series of a drug combination mixed at 1:1 ratio of the EC₅₀s of each drug were compared with single drug dilution series using the Chou-Talaly method.
FIG. 23. SHetA2, but not PRIMA-1, induces expression of the p53-regulated gene p21. qRT-PCR of mRNA isoloated from SKOV3 parental and stably-transfected p53 R273H mutant cells treated with solvent only, 20 µM PRIMA-1, 10 µM SHetA2 or the combination of 20 µM PRIMA-1 + 10 µM SHetA2. Expression values were normalized to GAPDH, and then the treated were normalized to the solvent only control samples.
FIG. 24. SHetA2, but not PRIMA-1, induces expression of the p53-regulated protein p21, while both drugs induce SLC7A11 and PARP cleavage in A2780 (wild type p53) ovarian cancer cells. Western blots of p21, SLC7A11 and PARP in the indicated cell lines after treatment with either SHetA2 or PRIMA-1 or both combined at the indicated concentrations.
FIG. 25. SHetA2, but not PRIMA-1, induces expression of the p53-regulated protein p21, while both drugs induce SLC7A11 and PARP cleavage in SKOV3 (p53 null) ovarian cancer cells. Western blots of p21, SLC7A11 and PARP in the indicated cell lines after treatment with either SHetA2 or PRIMA-1 or both combined at the indicated concentrations.
FIG. 26. SHetA2, but not PRIMA-1, induces expression of the p53-regulated protein p21, while both drugs induce SLC7A11 and PARP cleavage in SKOV3 R273H (R273H p53) ovarian cancer cells. Western blots of p21, SLC7A11 and PARP in the indicated cell lines after treatment with either SHetA2 or PRIMA-1 or both combined at the indicated concentrations.
FIG. 27. SHetA2, but not PRIMA-1, induces expression of the p53-regulated protein p21, while both drugs induce SLC7A11 and PARP cleavage in SKOV3 R248w (R248W p53) ovarian cancer cells. Western blots of p21, SLC7A11 and PARP in the indicated cell lines after treatment with either SHetA2 or PRIMA-1 or both combined at the indicated concentrations.
FIG. 28. Inhibition of caspase 3 reduces PARP-1 cleavage in A2780 (wild type p53) ovarian cancer cells. Western blots of p53, PARP-1 and caspase 3 in cultures with SHetA2 and/or PRIMA-1^{MET} in the absence or presence of the caspase 3 inhibitor Z-DEVD-FMK.
FIG. 29. Inhibition of caspase 3 reduces PARP-1 cleavage in MES-OV (R282W p53) ovarian cancer cells. Western blots of p53, PARP-1 and caspase 3 in cultures with SHetA2 and/or PRIMA-1^{MET} in the absence or presence of the caspase 3 inhibitor Z-DEVD-FMK.
FIG. 30. Inhibition of caspase 3 reduces PARP-1 cleavage in SKOV3 (p53 null) ovarian cancer cells. Western blots of p53, PARP-1 and caspase 3 in cultures with SHetA2 and/or PRIMA-1^{MET} in the absence or presence of the caspase 3 inhibitor Z-DEVD-FMK.
FIG. 31. Inhibition of caspase 3 reduces PARP-1 cleavage in SKOV3 R273H (R273H p53) ovarian cancer cells. Western blots of p53, PARP-1 and caspase 3 in cultures with SHetA2 and/or PRIMA-1^{MET} in the absence or presence of the caspase 3 inhibitor Z-DEVD-FMK.
FIG. 32. Increased sensitivity of SKOV3 cells is associated with reduced expression of SLC7A11. Bar graphs SLC7A11 band denisities normalized to the loading control (GAPDH) in Figs. 25-27 are compared. The sensitivies of the cell lines to PRIMA-1 as shown in FIG. 14 are SKOV3 R248W>SKOV3 R273H>SKOV3 parental.
FIG. 33. Combination of SHetA2 and PRIMA-1 increases reactive oxygen species (ROS) induction in comparison the induction by either drug alone in SKOV3 (p53 null) ovarian cancer cells. ROS levels in were measured after treatment with SHetA2 or PRIMA-1 alone or in combinations **p ≤ 0.01, ***p ≤ 0.001 ****p ≤ 0.0001.
FIG. 34. Combination of SHetA2 and PRIMA-1 increases ROS induction in comparison the induction by either drug alone in SKOV3 R273H (R273H p53) ovarian cancer cells. ROS levels were measured after treatment with SHetA2 or PRIMA-1 alone or in combinations **p ≤ 0.01, ***p ≤ 0.001 ****p ≤ 0.0001.
FIG. 35. Combination of SHetA2 and PRIMA-1 increases ROS induction in comparison the induction by either drug alone in SKOV3 R248W (R248W p53) ovarian cancer cells. ROS levels were measured after treatment with SHetA2 or PRIMA-1 alone or in combinations **p ≤ 0.01, ***p ≤ 0.001 ****p ≤ 0.0001.
FIG. 36. SKOV3 ovarian cancer cell lines expressing p53 mutants have significantly higher ROS/ATP ratios in comparison to the parental p53 null line and in association with sensitivity to PRIMA-1. Bar graphs of the ratio of ROS/ATP (each normalized to protein concentration). ANOVA with Tukey's multiple comparison test: *p ≤ 0.05, **p ≤ 0.01, ****p ≤ 0.0001. The sensitivities of the cell lines to PRIMA-1 as shown in FIG. 14 are SKOV3 R248W>SKOV3 R273H>SKOV3 parental p53 null cells.
FIG. 37. Combination of SHetA2 and PRIMA-1^{MET} significantly increases the ROS/ATP ratio over the elevated ratio caused by either drug alone in A2780 (wild type p53) ovarian cancer cells. Ratio of ROS/ATP in A2780 cells after treatment with SHetA2 or PRIMA-1^{MET} or both combined.
FIG. 38. Reduction of p53 significantly decreased sensitivity of A2780 (wild type p53) ovarian cancer cells to ATP depletion caused by SHetA2 and PRIMA-1^{MET} combination treatment. ATP concentrations were measured in A2780 cells stably transfected with control shRNA or p53 shRNA after treatment with SHetA2 and PRIMA-1^{MET} drug combination.
FIG. 39. SHetA2 or PRIMA-1^{MET} had no significant effects on body weight or ascites (measured by abdominal size) in nu/nu immunocompromised mice injected intraperitoneally with MESOV3 ovarian cancer cells. Mice body weights were measured during the duration of treatment duration and abdominal size was measured at the end of the treatment period. ANOVA: ** p<0.01 for the untreaed control weight compared to the other groups (likely due to cancer-induced cachexia).
FIG. 40. Fewer tumors developed in intraperitoneally MESOV ovarian cancer cell-injected mice treated with SHetA2 and/or PRIMA-1^{MET}. Photos of peritoneal tumors harvested from MESOV ovarian cancer cell - injected athymic nude mice after the treatment period.
FIG 41. SHetA2 and PRIMA-1^{MET} significantly increased in an additive manner the tumor free rates in nu/nu mice injected intraperitoneally with MESOV ovarian cancer cells. Tumor free rates in the various treatment groups, Linear Regression Model: SHetA2 (p=0.004, OR=10.384, 95% CI: 2.158, 48.965) and PRIMA1^{MET} (p=0.048, OR=4.464, 95% CI: 1.014, 19.655) functioned additively in preventing tumor development.
FIG. 42. SHetA2 or PRIMA-1^{MET} did not cause kidney or liver toxicity in nu/nu mice injected intraperitoneally with MESOV ovarian cancer cells. 20X images of H & E staining of liver and kidney specimen of mice tumor model to determine toxicity of drug combination. The kidneys exhibited glomeruli that were well formed without inflammation and the tubules were normal with no necrosis, apoptosis or inflammation. Livers exhibited no necrosis, fat deposition or inflammation.
FIG. 43A. Combination of SHetA2 and PRIMA-1^{MET} significantly increased SLC7A11 in tumors of nu/nu mice injected intraperitoneally with MESOV ovarian cancer cells. Western blot analysis was performed on tumors from three randomly chosen mice in each treatment group to analyze expression of Pax8 (marker of ovarian cancer), SLC7A11 (marker of PRIMA-1/PRIMA-1^{MET} sensitivity) and p53 in mice tumor specimens.
FIG. 43B. Combination of SHetA2 and PRIMA-1^{MET} significantly increased SLC7A11 in tumors of nu/nu mice injected intraperitoneally with MESOV ovarian cancer cells. Bar graph of p53 and SLC7A11 band densities normalized to the loading controls in the western blot of Fig 43A.. ANOVA: *p<0.05
FIG. 44. Isobologram of SHetA2 and palbociclib combination in SiHa cervical cancer cells (human papillomavirus positive). Fold effects of a 2-fold dilution series of a drug combination mixed at 1:1 ratio of the EC₅₀s of each drug were compared with single drug dilution series using the Chou-Talaly method.
FIG. 45. Isobologram of SHetA2 and palbociclib combination in Caski cervical cancer cells (human papillomavirus positive). Fold effects of a 2-fold dilution series of a drug combination mixed at 1:1 ratio of the EC₅₀s of each drug were compared with single drug dilution series using the Chou-Talaly method.
FIG. 46. Isobologram of SHetA2 and palbociclib combination in C33a cervical cancer cells (human papillomavirus negative). Fold effects of a 2-fold dilution series of a drug combination mixed at 1:1 ratio of the EC₅₀s of each drug were compared with single drug dilution series using the Chou-Talaly method.
FIG. 47. Cell Death Induction by SHetA2 and Palbociclib. Photomicrographs of SiHa cultures treated for 24 hours with each drug alone and in combination as indicated. Rounding up of the cells indicates cell death.
FIG. 48. Cell Death Induction by SHetA2 and Palbociclib. Photomicrographs of C33A cultures treated for 24 hours with each drug alone and in combination as indicated. Rounding up of the cells indicates cell death.

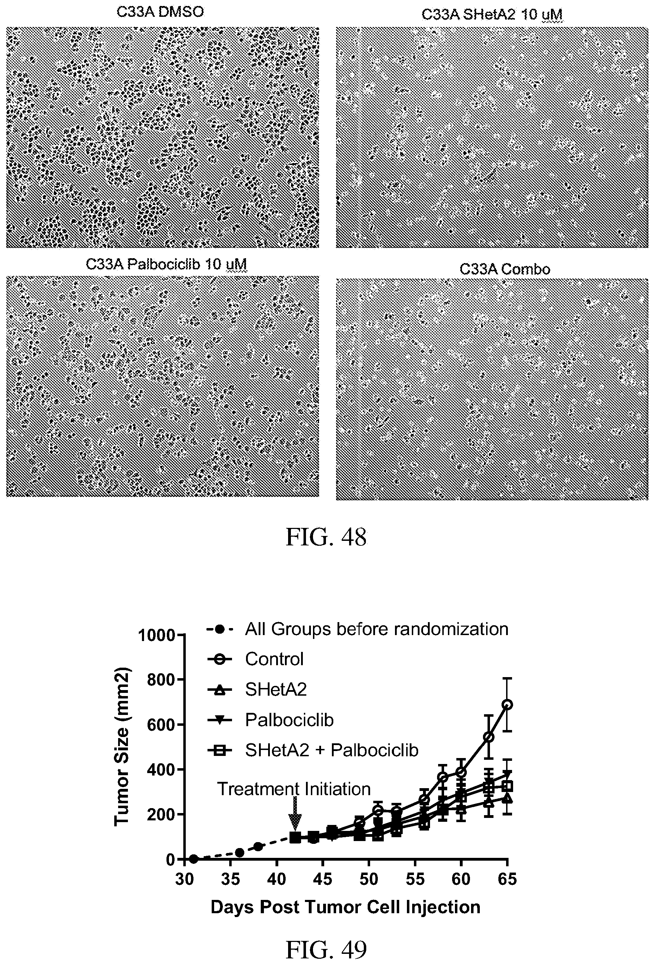

FIG. 49. SHetA2 and palbociclib significantly reduce SiHa cervical cancer cell line xenograft tumor growth in an additive manner. Immunocompromized mice bearing SiHa xenografts were gavaged with 60 mg/kg SHetA2, 100 mg/kg palbociclib or the combination every other day. Controls were gavaged with the same Kolliphor HS15 used to suspend the drugs. Using a generalized estimating equations (GEE) model to analyze tumor growth treated as a continuous variable, there was a significant additive effect of SHetA2 (p<.001) and palbociclib (p<.001) on day 65 of treatment. This additive effect between SHetA2 (p=.001) and palbociclib (p=0.051) was trending significant at day 63 of treatment. Additionally, SHetA2 starts to show significant tumor suppressing activity as early as day 63 of treatment (p=0.045).

### DETAILED DESCRIPTION

Many cancer deaths, including most deaths due to ovarian cancer, are caused by disease recurrence. Current maintenance therapies to reduce recurrence of cancer are often limited by toxicity and no proven effect on overall survival. The present disclosure is directed to synergistically-effective drug combinations comprising a heteroarotinoid (SHetA2), and at least one of an Azabicyclooctan-3-one derivative (PRIMA-1 or PRIMA^{MET}), and a CDK4/6 inhibitor (Palbociclib, Abemaciclib or Ribociclib) which have a synergistic effect in causing cancer cell death, reduction of tumor size, reduction of tumor growth, inhibition of cancer metastases, and/or inhibition of tumor recurrence. The present work demonstrated, for example, that SHetA2, a mortalin-targeting drug, and PRIMA-1^{MET}, a p53 reactivator drug, inhibit ovarian cancer cell line growth synergistically, thus can be used in combination for ovarian cancer treatment and/or maintenance therapy (to prevent recurrence), as well as for treatment and/or maintenance therapy of other cancers.

The examples described below, which include particular embodiments, will serve to illustrate the practice of the present disclosure, it being understood that the particulars shown are by way of example and for purposes of illustrative discussion of particular embodiments only and are presented in the cause of providing what is believed to be a useful and readily understood description of procedures as well as of the principles and conceptual aspects of the inventive concepts.

### List of Abbreviations

- CI: Combination index
- DRI: Dose-reduction index
- FDA: Food and Drug Administration
- GOG: Gynecologic Oncology Group
- HGSOC: High-grade serous ovarian cancer
- hFTSECs: Human fallopian tube Secretory epithelial cells
- IC: Inhibitory concentration
- IC50: Half maximal inhibitory concentration
- M-p53: Missense mutant p53
- MTT: 3-(4,5-dimethylthiazol-2-yl)-2,5-diphenyltetrazolium bromide
- P53: Tumor protein 53
- PARP: Poly ADP ribose polymerase
- PRIMA-1: 2,2-Bishydroxymethyl-1-azabicyclo[2.2.2]octan-3-one
- PRIMA-1^{MET}: 2-(hydroxymethyl)-2-(methoxymethyl)-1-azabicyclo [2.2.2]octan-3-one
- RAID: Rapid Access to Intervention Development
- RAPID: Rapid Access to Preventive Intervention Development
- SHetA2: [(4-nitrophenyl)amino][2,2,4,4-tetramethylthiochroman-6-yl]amino] methane-thione
- TCGA: The Cancer Genome Atlas
- TMA: Tumor Microarray
- TP53: Tumor protein 53 gene
- OS: Overall Survival
- ROS: Reactive oxygen species

All patents, published patent applications, and non-patent publications mentioned in the specification are indicative of the level of skill of those skilled in the art to which the present disclosure pertains.

Unless otherwise defined herein, scientific and technical terms used in connection with the present disclosure shall have the meanings that are commonly understood by those having ordinary skill in the art. Further, unless otherwise required by context, singular terms shall include pluralities and plural terms shall include the singular.

As utilized in accordance with the methods and compositions of the present disclosure, the following terms, unless otherwise indicated, shall be understood to have the following meanings:
The use of the word "a" or "an" when used in conjunction with the term "comprising" in the claims and/or the specification may mean "one," but it is also consistent with the meaning of "one or more," "at least one," and "one or more than one." The use of the term "or" in the claims is used to mean "and/or" unless explicitly indicated to refer to alternatives only or when the alternatives are mutually exclusive, although the disclosure supports a definition that refers to only alternatives and "and/or." The use of the term "at least one" will be understood to include one as well as any quantity more than one, including but not limited to, 2, 3, 4, 5, 6, 7, 8, 9, 10, 15, 20, 30, 40, 50, 100, or any integer inclusive therein. The term "at least one" may extend up to 100 or 1000 or more, depending on the term to which it is attached; in addition, the quantities of 100/1000 are not to be considered limiting, as higher limits may also produce satisfactory results. In addition, the use of the term "at least one of X, Y and Z" will be understood to include X alone, Y alone, and Z alone, as well as any combination of X, Y and Z.

As used in this specification and claims, the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

The term "or combinations thereof" as used herein refers to all permutations and combinations of the listed items preceding the term. For example, "A, B, C, or combinations thereof" is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, AAB, BBC, AAABCCCC, CBBAAA, CABABB, and so forth. The skilled artisan will understand that typically there is no limit on the number of items or terms in any combination, unless otherwise apparent from the context.

Throughout this application, the terms "about" or "approximately" are used to indicate that a value includes the inherent variation of error for the composition, the method used to administer the composition, or the variation that exists among the study subjects. As used herein the qualifiers "about" or "approximately" are intended to include not only the exact value, amount, degree, orientation, or other qualified characteristic or value, but are intended to include some slight variations due to measuring error, manufacturing tolerances, observer error, and combinations thereof, for example. The term "about" or "approximately", where used herein when referring to a measurable value such as an amount, a temporal duration, and the like, is meant to encompass, for example, variations of ± 20% or ± 10%, or ± 5%, or ± 1%, or ± 0.1% from the specified value, as such variations are appropriate to perform the disclosed methods and as understood by persons having ordinary skill in the art. As used herein, the term "substantially" means that the subsequently described event or circumstance completely occurs or that the subsequently described event or circumstance occurs to a great extent or degree. For example, the term "substantially" means that the subsequently described event or circumstance occurs at least 80% of the time, at least 90% of the time, at least 91% of the time, at least 92% of the time, at least 93% of the time, at least 94% of the time, at least 95% of the time, at least 96% of the time, at least 97% of the time, at least 98% of the time, or at least 99% of the time.

As used herein any reference to "one embodiment" or "an embodiment" means that a particular element, feature, composition, structure, or characteristic described in connection with the embodiment is included in at least one embodiment. The appearances of the phrase "in one embodiment" in various places in the specification are not necessarily all referring to the same embodiment.

The term "mutant" or "variant" is intended to refer to a protein, peptide, nucleic acid or organism which has at least one amino acid or nucleotide which is different from the wild type version of the protein, peptide, nucleic acid, or organism and includes, but is not limited to, point substitutions, multiple contiguous or non-contiguous substitutions, chimeras, or fusion proteins, and the nucleic acids which encode them. Examples of conservative amino acid substitutions include, but are not limited to, substitutions made within the same group such as within the group of basic amino acids (such as arginine, lysine, histidine), acidic amino acids (such as glutamic acid and aspartic acid), polar amino acids (such as glutamine and asparagine), hydrophobic amino acids (such as leucine, isoleucine, and valine), aromatic amino acids (such as phenylalanine, tryptophan, tyrosine) and small amino acids (such as glycine, alanine, serine, threonine, methionine). Other examples of possible substitutions are described below.

The term "pharmaceutically acceptable" refers to compounds and compositions which are suitable for administration to humans and/or animals without undue adverse side effects such as toxicity, irritation and/or allergic response commensurate with a reasonable benefit/risk ratio.

By "biologically active" is meant the ability to modify the physiological system of an organism without reference to how the active agent has its physiological effects.

As used herein, "pure," or "substantially pure" means an object species is the predominant species present (i.e., on a molar basis it is more abundant than any other object species in the composition thereof), and particularly a substantially purified fraction is a composition wherein the object species comprises at least about 50 percent (on a molar basis) of all macromolecular species present. Generally, a substantially pure composition will comprise more than about 80% of all macromolecular species present in the composition, more particularly more than about 85%, more than about 90%, more than about 95%, or more than about 99%. The term "pure" or "substantially pure" also refers to preparations where the object species (e.g., the active agent) is at least 60% (w/w) pure, or at least 70% (w/w) pure, or at least 75% (w/w) pure, or at least 80% (w/w) pure, or at least 85% (w/w) pure, or at least 90% (w/w) pure, or at least 92% (w/w) pure, or at least 95% (w/w) pure, or at least 96% (w/w) pure, or at least 97% (w/w) pure, or at least 98% (w/w) pure, or at least 99% (w/w) pure, or 100% (w/w) pure.

The terms "subject" and "patient" are used interchangeably herein and will be understood to refer to a warm blooded animal, particularly a mammal. Non-limiting examples of animals within the scope and meaning of this term include dogs, cats, rabbits, rats, mice, guinea pigs, chinchillas, hamsters, ferrets, horses, pigs, goats, cattle, sheep, zoo animals, camels, llamas, non-human primates, including Old and New World monkeys and non-human primates (e.g., cynomolgus macaques, chimpanzees, rhesus monkeys, orangutans, and baboons), and humans.

Where used herein the term "active agent" refers to a compound or composition having biological activity.

Therapeutic drug combinations of the present disclosure include a first active agent which is SHetA2; and a second active agent selected from the group consisting of PRIMA-1, PRIMA-1^{MET}, Palbociclib, Abemaciclib, and Ribociclib.

Where used herein the terms heteroarotinoid, Azabicyclooctan-3-one derivative, and CDK4/6 inhibitor, and specific examples thereof, are intended to include pharmaceutically-acceptable salts, hydrates, solvates, and amorphous solids thereof.

Where used herein "PRIMA-1/PRIMA-1^{MET}" refers to either PRIMA-1 or PRIMA-1^{MET}.

"Treatment" refers to therapeutic treatments. "Prevention" refers to prophylactic or preventative treatment measures. The term "treating" refers to administering the composition to a patient for therapeutic purposes.

The terms "therapeutic composition" and "pharmaceutical composition" refer to an active agent-containing composition that may be administered to a subject by any method known in the art or otherwise contemplated herein, wherein administration of the composition brings about a therapeutic effect as described elsewhere herein. In addition, the compositions of the present disclosure may be designed to provide delayed, controlled, extended, and/or sustained release using formulation techniques which are well known in the art.

The term "effective amount" refers to an amount of an active agent which is sufficient to exhibit a detectable therapeutic effect without excessive adverse side effects (such as toxicity, irritation and allergic response) commensurate with a reasonable benefit/risk ratio when used in the manner of the inventive concepts. The effective amount for a patient will depend upon the type of patient, the patient's size and health, the nature and severity of the condition to be treated, the method of administration, the duration of treatment, the nature of concurrent therapy (if any), the specific formulations employed, and the like. Thus, it is not possible to specify an exact effective amount in advance. However, the effective amount for a given situation can be determined by one of ordinary skill in the art using routine experimentation based on the information provided herein.

The term "ameliorate" means a detectable or measurable improvement in a subject's condition, disease or symptom thereof. A detectable or measurable improvement includes a subjective or objective decrease, reduction, inhibition, suppression, limit or control in the occurrence, frequency, severity, progression, or duration of the condition or disease, or an improvement in a symptom or an underlying cause or a consequence of the disease, or a reversal of the disease. A successful treatment outcome can lead to a "therapeutic effect," or "benefit" of ameliorating, decreasing, reducing, inhibiting, suppressing, limiting, controlling or preventing the occurrence, frequency, severity, progression, or duration of a disease or condition, or consequences of the disease or condition in a subject.

A decrease or reduction in worsening, such as stabilizing the condition or disease, is also a successful treatment outcome. A therapeutic benefit therefore need not be complete ablation or reversal of the disease or condition, or any one, most or all adverse symptoms, complications, consequences or underlying causes associated with the disease or condition. Thus, a satisfactory endpoint may be achieved when there is an incremental improvement such as a partial decrease, reduction, inhibition, suppression, limit, control, or prevention in the occurrence, frequency, severity, progression, or duration, or inhibition or reversal of the condition or disease (e.g., stabilizing), over a short or long duration of time (hours, days, weeks, months, etc.). Effectiveness of a method or use, such as a treatment that provides a potential therapeutic benefit or improvement of a condition or disease, can be ascertained by various methods and testing assays.

Where used herein the term cancer, cancer cell, or tumor, can be used in reference to various cancers including, but not limited to ovarian cancer, cervical cancer, peritoneal cancer, uterine cancer, vulvar cancer, oral cancer, pharyngeal cancer, oropharyngeal cancer, nasopharyngeal cancer, respiratory cancer, urogenital cancer, gastrointestinal cancer, central or peripheral nervous system tissue cancer, an endocrine cancer, neuroendocrine cancer, glioma, carcinoma, lymphoma, melanoma, fibroma, meningioma, brain cancer, renal cancer, pheochromocytoma, pancreatic islet cell cancer, Li-Fraumeni tumors, thyroid cancer, parathyroid cancer, pituitary tumors, adrenal gland tumors, osteogenic sarcoma tumors, multiple neuroendocrine type I and type II tumors, head and neck cancer, prostate cancer, esophageal cancer, tracheal cancer, liver cancer, bladder cancer, stomach cancer, pancreatic cancer, uterine cancer, testicular cancer, colon cancer, rectal cancer, and skin cancer, and any other cancer which comprises one or more mutations in TP53 gene or which is driven by cyclin D1/cdk4/6 complex.

In other embodiments, the term cancer, cancer cell, or tumor, can be used in reference to various cancers including, but not limited to breast cancer (e.g., estrogen receptor positive or negative, progesterone receptor positive or negative, HER-2 positive or negative, triple-negative breast cancer, or BRCA1 and/or BRCA2 positive or negative), lung cancer (e.g., non-small cell lung cancer and small cell lung cancer), endometrial cancer, glioblastoma, B-cell malignancies, biliary tract cancer, bone cancer, choriocarcinoma, connective tissue cancer, cancers of the digestive system, gallbladder cancer, hepatocellular carcinoma, intra-epithelial neoplasm, kidney cancer, liver cancer, lymphoma, skin cancer (e.g., melanoma and basal cell carcinoma), neuroblastoma, mesothelioma, neuroglioma, oral cavity cancer, pediatric cancer, pancreatic endocrine tumors, pituitary adenoma, thymoma, renal cell carcinoma, salivary gland cancer, sarcoma (e.g., Ewing's sarcoma, fibrosarcoma, and rhabdomyosarcoma), small bowel cancer, ureteral cancer, cancers of the urinary system, and hematological cancers (e.g., acute myeloid leukemia and multiple myeloma).

The active agents of the present disclosure may be present in the pharmaceutical compositions (alone or in combination) at any concentration that allows the pharmaceutical composition to function in accordance with the present disclosure; for example, but not by way of limitation, the compound(s) may be present in a carrier, diluent, or buffer solution in a wt/wt or vol/vol range having a lower level selected from 0.00001%, 0.0001%, 0.005%, 0.001%, 0.005%, 0.01%, 0.05%, 0.1%, 0.2%, 0.3%, 0.4%, 0.5%, 0.6%, 0.7%, 0.8%, 0.9%, 1.0%, 1.1%, 1.2%, 1.3%, 1.4%, 1.5%, 1.6%, 1.7%, 1.8%, 1.9% and 2.0%; and an upper level selected from 3%, 3.5%, 4%, 4.5%, 5%, 5.5%, 6%, 6.5%, 7%, 7.5%, 8%, 8.5%, 9%, 9.5%, 10%, 11%, 12%, 13%, 14%, 15%, 16%, 17%, 18%, 19%, 20%, 25%, 30%, 35%, 40%, 45%, 50%, 55%, 60%, 65%, 70%, 75%, 80%, 85%, 90%, and 95%. Non-limiting examples of particular wt/wt or vol/vol ranges include a range of from about 0.0001% to about 95%, a range of from about 0.001% to about 75%; a range of from about 0.005% to about 50%; a range of from about 0.01% to about 40%; a range of from about 0.05% to about 35%; a range of from about 0.1% to about 30%; a range of from about 0.1% to about 25%; a range of from about 0.1% to about 20%; a range of from about 1% to about 15%; a range of from about 2% to about 12%; a range of from about 5% to about 10%; and the like. Any other range that includes a lower level selected from the above-listed lower level concentrations and an upper level selected from the above-listed upper level concentrations also falls within the scope of the present disclosure. Percentages used herein may be weight percentages (wt%) or volume percentages (vol%).

In certain non-limiting embodiments, an effective amount or therapeutic dosage of a pharmaceutical composition of the present disclosure contains, sufficient active agent to deliver from about 0.001 µg/kg to about 100 mg/kg (weight of active agent/body weight of the subject). For example, the composition will deliver about 0.01 µg/kg to about 50 mg/kg, and more particularly about 0.1 µg/kg to about 10 mg/kg, and more particularly about 1 µg/kg to about 1 mg/kg. Practice of a method of the present disclosure may comprise administering to a subject an effective amount of the active agent in any suitable systemic and/or local formulation, in an amount effective to deliver the therapeutic dosage of the active agent. In certain embodiments, an effective dosage may be, in a range of about 1 µg/kg to about 1 mg/kg of the active agent.

In certain non-limiting embodiments, an effective amount or therapeutic dosage of a pharmaceutical composition of the present disclosure contains a heteroarotinoid (SHetA2) in a dose range that will result in 0.01 micromolar to 100 millimolar blood or tissue levels, and a Azabicyclooctan-3-one derivative, and/or CDK4/6 inhibitor in a concentration in a dose range that will result in 0.01 micromolar to 100 millimolar blood or tissue levels. In certain non-limiting embodiments, an effective amount or therapeutic dosage of a pharmaceutical composition of the present disclosure contains SHetA2 in a concentration in a dose range that will result in 0.01 micromolar to 100 millimolar, and a second active agent (e.g., Palbociclib) in a dose range that will result in 0.01 micromolar to 100 millimolar blood or tissue levels.

In non-limiting embodiments, the synergistic ratio of the compositions of the first active agent and the second active agent (e.g., SHetA2:PRIMA-1, SHetA2:PRIMA-1^{MET}, or SHetA2: Palbociclib, etc.) may be in a range from 50:1 wt/wt to 1:50 wt/wt, 45:1 wt/wt to 1:45 wt/wt, 40:1 wt/wt to 1:40 wt/wt, 35:1 wt/wt to 1:35 wt/wt, 30:1 wt/wt to 1:30 wt/wt, 25:1wt/wt to 1:25 wt/wt, e.g., 25:1, 24:1, 23:1, 22:1, 21:1, 20:1, 19:1, 18:1, 17:1, 16:1, 15:1, 14:1, 13:1, 12:1, 11:1, 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, 1:10, 1:11, 1:12, 1:13, 1:14, 1:15, 1:16, 1:17, 1:18, 1:19, 1:20, 1:21, 1:22, 1:23, 1:24, or 1:25 or may be in any range bounded by the ratios listed above.

Practice of the present disclosure may comprise administering to a subject therapeutically effective amounts of the active agents in any suitable systemic and/or local formulation, in an amount effective to deliver the dosages listed herein. The dosage can be administered, for example but not by way of limitation, on a one-time basis, or administered at multiple times (for example but not by way of limitation, from one to five times per day, or once or twice per week), or continuously via a venous drip, depending on the desired therapeutic effect. In one non-limiting example of a therapeutic method of the present disclosure, the active agent is provided in an IV infusion in the range of from about 0.01 mg/kg to about 10 mg/kg of body weight once a day.

The term "synergistic" or "synergistic effect" or "synergistic interaction" as used herein refers to a therapeutic combination which is more effective than the additive effects of the two or more single active agents. A "synergistic ratio" is a ratio of two compounds which results in a synergistic effect. A determination of a synergistic interaction between the active agents described herein may be based on the results obtained from the assays described herein. The results of these assays can be analyzed using the Chou and Talalay combination method (Chou TC, Talalay P. "Quantitative analysis of dose-effect relationships: the combined effects of multiple drugs or enzyme inhibitors." Adv Enzyme Regul. 1984;22: 27-55) and Dose-Effect Analysis with CalcuSyn software in order to obtain a Combination Index. The combinations provided herein have been evaluated in several assay systems, and the data can be analyzed utilizing a standard program for quantifying synergism, additivism, and antagonism among anticanceragents. An example program is that described by Chou and Talalay, in "New Avenues in Developmental Cancer Chemotherapy," Academic Press, 1987, Chapter 2. Combination Index values less than 0.9 indicate synergy, values greater than 1.2 indicate antagonism and values between 0.9 to 1.1 indicate additive effects (e.g., see Table 4 below). The combination therapy may provide "synergy" and prove "synergistic", i.e., the effect achieved when the active agents used together is greater than the sum of the effects that results from using the compounds separately. A synergistic effect may be attained when the active agents are: (1) co-formulated and administered or delivered simultaneously in a combined, unit dosage formulation; (2) delivered in succession ("alternation therapy") or in parallel as separate formulations; or (3) by some other effective regimen. When delivered in successive administrations, a synergistic effect may be attained when the compounds are administered or delivered sequentially, e.g., by different injections in separate syringes. In general, during alternation therapy, an effective dosage of each active ingredient is administered sequentially, i.e., serially, whereas in combination therapy, effective dosages of two or more active ingredients are administered together.

The active agents of the combination therapies of the present disclosure may be used conjointly. As used herein the terms "conjointly" or "conjoint administration" refers to any form of administration of two or more different therapeutic compounds (i.e., active agents) such that the second compound is administered while the previously administered therapeutic compound is still effective in the body, whereby the two or more compounds are simultaneously active in the patient, enabling a synergistic interaction of the compounds. For example, the different therapeutic compounds can be administered either in the same formulation, or in separate formulations, either concomitantly (together) or sequentially. When administered sequentially the different compounds may be administered immediately in succession, or separated by a suitable duration of time, as long as the active agents function together in a synergistic manner. In certain embodiments, the different therapeutic compounds can be administered within one hour of each other, within two hours of each other, within 3 hours of each other, within 6 hours of each other, within 12 hours of each other, within 24 hours of each other, within 36 hours of each other, within 48 hours of each other, within 72 hours of each other, or more. Thus an individual who receives such treatment can benefit from a combined effect of the different therapeutic compounds.

The active agents of the present disclosure can be administered to a subject by any of a number of effective routes of administration including, for example, orally (for example, drenches as in aqueous or non-aqueous solutions or suspensions, tablets, capsules (including sprinkle capsules and gelatin capsules), boluses, powders, granules, pastes for application to the tongue); absorption through the oral mucosa (e.g., sublingually); anally, rectally or vaginally (for example, as a pessary, cream or foam); parenterally (including intramuscularly, intravenously, subcutaneously or intrathecally as, for example, a sterile solution or suspension); nasally; intraperitoneally; subcutaneously; transdermally (for example as a patch applied to the skin); and topically (for example, as a cream, ointment or spray applied to the skin, or as an eye drop). The compounds may also be formulated for inhalation. In certain embodiments, a compound may be simply dissolved or suspended in sterile water. Oral formulations may be formulated such that the active agents passes through a portion of the digestive system before being released, for example it may not be released until reaching the small intestine, or the colon. Details of appropriate routes of administration and compositions suitable for same can be found in, for example, U.S. Pat. Nos. 6,110,973, 5,763,493, 5,731,000, 5,541,231, 5,427,798, 5,358,970 and 4,172,896, as well as in patents cited therein.

Tablets, and other solid dosage forms of the pharmaceutical compositions, such as dragees, capsules (including sprinkle capsules and gelatin capsules), pills and granules, may optionally be scored or prepared with coatings and shells, such as enteric coatings and other coatings well known in the pharmaceutical- formulating art. They may also be formulated so as to provide slow or controlled release of the active ingredient therein using, for example, hydroxypropylmethyl cellulose in varying proportions to provide the desired release profile, other polymer matrices, liposomes and/or microspheres. They may be sterilized by, for example, filtration through a bacteria-retaining filter, or by incorporating sterilizing agents in the form of sterile solid compositions that can be dissolved in sterile water, or some other sterile injectable medium immediately before use. These compositions may also optionally contain opacifying agents and may be of a composition that they release the active ingredient(s) only, or preferentially, in a certain portion of the gastrointestinal tract, optionally, in a delayed manner. Examples of embedding compositions that can be used include polymeric substances and waxes. The active ingredient can also be in micro-encapsulated form, if appropriate, with one or more of the above-described excipients.

Liquid dosage forms useful for oral administration include pharmaceutically acceptable emulsions, lyophiles for reconstitution, microemulsions, solutions, suspensions, syrups and elixirs. In addition to the active ingredient, the liquid dosage forms may contain inert diluents commonly used in the art, such as, for example, water or other solvents, cyclodextrins and derivatives thereof, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, coloring, perfuming and preservative agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Formulations of the pharmaceutical compositions for rectal, vaginal, or urethral administration may be presented as a suppository, which may be prepared by mixing one or more active compounds with one or more suitable nonirritating excipients or carriers comprising, for example, cocoa butter, polyethylene glycol, a suppository wax or a salicylate, and which is solid at room temperature, but liquid at body temperature and, therefore, will melt in the rectum or vaginal cavity and release the active compound.

Formulations which are suitable for vaginal administration also include pessaries, tampons, creams, gels, pastes, foams or spray formulations containing such carriers as are known in the art to be appropriate. In certain embodiments, the active agents of the present disclosure can be formulated into suppositories, slow release formulations, or intrauterine delivery devices (IUDs).

Formulations of the pharmaceutical compositions for administration to the mouth may be presented as a mouthwash, or an oral spray, or an oral ointment.

Alternatively or additionally, compositions can be formulated for delivery via a catheter, stent, wire, or other intraluminal device. Delivery via such devices may be especially useful for delivery to the bladder, urethra, ureter, rectum, or intestine.

Dosage forms for the topical or transdermal administration include powders, sprays, ointments, pastes, creams, lotions, gels, solutions, patches and inhalants. The active compound may be mixed under sterile conditions with a pharmaceutically acceptable carrier, and with any preservatives, buffers, or propellants that may be required. The ointments, pastes, creams and gels may contain, in addition to an active compound, excipients, such as animal and vegetable fats, oils, waxes, paraffins, starch, tragacanth, cellulose derivatives, polyethylene glycols, silicones, bentonites, silicic acid, talc and zinc oxide, or mixtures thereof. Powders and sprays can contain, in addition to an active compound, excipients such as lactose, talc, silicic acid, aluminum hydroxide, calcium silicates and polyamide powder, or mixtures of these substances. Sprays can additionally contain customary propellants, such as chlorofluorohydrocarbons and volatileunsubstituted hydrocarbons, such as butane and propane. Transdermal patches have the added advantage of providing controlled delivery of a compound of the present invention to the body. Such dosage forms can be made by dissolving or dispersing the active compound in the proper medium. Absorption enhancers can also be used to increase the flux of the compound across the skin. The rate of such flux can be controlled by either providing a rate controlling membrane or dispersing the compound in a polymer matrix or gel.

Ophthalmic formulations, eye ointments, powders, solutions and the like, are also contemplated as being within the scope of this invention. Exemplary ophthalmic formulations are described in U.S. Publication Nos. 2005/0080056, 2005/0059744, 2005/0031697 and 2005/004074 and U.S. Pat. No. 6,583,124. If desired, liquid ophthalmic formulations have properties similar to that of lacrimal fluids, aqueous humor or vitreous humor or are compatable with such fluids. A preferred route of administration is local administration (e.g., topical administration, such as eye drops, or administration via an implant).

The phrases "parenteral administration" and "administered parenterally" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal and intrasternal injection and infusion.

Pharmaceutical compositions suitable for parenteral administration comprise one or more active compounds in combination with one or more pharmaceutically acceptable sterile isotonic aqueous or nonaqueous solutions, dispersions, suspensions or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents.

Examples of suitable aqueous and nonaqueous carriers that may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents that delay absorption such as aluminum monostearate and gelatin.

In some cases, in order to prolong the effect of a drug, it is desirable to slow the absorption of the drug from subcutaneous or intramuscular injection. This may be accomplished by the use of a liquid suspension of crystalline or amorphous material having poor water solubility. The rate of absorption of the drug then depends upon its rate of dissolution, which, in turn, may depend upon crystal size and crystalline form. Alternatively, delayed absorption of a parenterally administered drug form is accomplished by dissolving or suspending the drug in an oil vehicle.

Injectable depot forms are made by forming microencapsulated matrices of the subject compounds in biodegradable polymers such as polylactide-polyglycolide. Depending on the ratio of drug to polymer, and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions that are compatible with body tissue.

As noted, effective amounts of the active agents may be administered orally, in the form of a solid or liquid preparations such as capsules, pills, tablets, lozenges, melts, powders, suspensions, solutions, elixirs or emulsions. Solid unit dosage forms can be capsules of the ordinary gelatin type containing, for example, surfactants, lubricants, and inert fillers such as lactose, sucrose, and cornstarch, or the dosage forms can be sustained release preparations. The pharmaceutical composition may contain a solid carrier, such as a gelatin or an adjuvant. The tablet, capsule, and powder may contain from about .05 to about 95% of the active substance compound by dry weight. When administered in liquid form, a liquid carrier such as water, petroleum, oils of animal or plant origin such as peanut oil, mineral oil, soybean oil, or sesame oil, or synthetic oils may be added. The liquid form of the pharmaceutical composition may further contain physiological saline solution, dextrose or other saccharide solution, or glycols such as ethylene glycol, propylene glycol, or polyethylene glycol. When administered in liquid form, the pharmaceutical composition particularly contains from about 0.005 to about 95% by weight of the active agent(s). For example, a dose of about 10 mg to about 1000 mg once or twice a day could be administered orally.

In another embodiment, the active agents of the present disclosure can be tableted with conventional tablet bases such as lactose, sucrose, and cornstarch in combination with binders, such as acacia, cornstarch, or gelatin, disintegrating agents such as potato starch or alginic acid, and a lubricant such as stearic acid or magnesium stearate. Liquid preparations are prepared by dissolving the active agents in an aqueous or non-aqueous pharmaceutically acceptable solvent which may also contain suspending agents, sweetening agents, flavoring agents, and preservative agents as are known in the art.

For parenteral administration, for example, the active agents may be dissolved in a physiologically acceptable pharmaceutical carrier and administered as either a solution or a suspension. Illustrative of suitable pharmaceutical carriers are water, saline, dextrose solutions, fructose solutions, ethanol, or oils of animal, vegetative, or synthetic origin. The pharmaceutical carrier may also contain preservatives and buffers as are known in the art.

When an effective amount of the active agents is administered by intravenous, cutaneous, or subcutaneous injection, the compound is particularly in the form of a pyrogen-free, parenterally acceptable aqueous solution or suspension. The preparation of such parenterally acceptable solutions, having due regard to pH, isotonicity, stability, and the like, is well within the skill in the art. A particular pharmaceutical composition for intravenous, cutaneous, or subcutaneous injection may contain, in addition to the active agent, an isotonic vehicle such as Sodium Chloride Injection, Ringer's Injection, Dextrose Injection, Dextrose and Sodium Chloride Injection, Lactated Ringer's Injection, or other vehicle as known in the art. The pharmaceutical compositions of the present disclosure may also contain stabilizers, preservatives, buffers, antioxidants, or other additives known to those of skill in the art.

As noted, particular amounts and modes of administration can be determined by one skilled in the art. One skilled in the art of preparing formulations can readily select the proper form and mode of administration, depending upon the particular characteristics of the active agents selected, the condition to be treated, the stage of the condition, and other relevant circumstances using formulation technology known in the art, described, for example, in Remington: The Science and Practice of Pharmacy, 22nd ed*.*

Additional pharmaceutical methods may be employed to control the duration of action of the active agents. Increased half-life and/or controlled release preparations may be achieved through the use of proteins or polymers to conjugate, complex with, and/or absorb the active agents as discussed previously herein. The controlled delivery and/or increased half-life may be achieved by selecting appropriate macromolecules (for example but not by way of limitation, polysaccharides, polyesters, polyamino acids, homopolymers, polyvinyl pyrrolidone, ethylenevinylacetate, methylcellulose, or carboxymethylcellulose, and acrylamides such as N-(2-hydroxypropyl) methacrylamide), and the appropriate concentration of macromolecules as well as the methods of incorporation, in order to control release.

Another possible method useful in controlling the duration of action of the active agents by controlled release preparations and half-life is incorporation of the active agents or their functional derivatives into particles of a polymeric material such as polyesters, polyamides, polyamino acids, hydrogels, poly(lactic acid), ethylene vinylacetate copolymers, copolymer micelles of, for example, polyethylene glycol (PEG) and poly(1-aspartamide).

Additional pharmaceutical methods may be employed to increase bioavailability of the drug, such as Kolliphor HS15.

It is also possible to entrap the active agents in microcapsules prepared, for example, by coacervation techniques or by interfacial polymerization (for example, hydroxymethylcellulose or gelatine-microcapsules and poly-(methylmethacylate) microcapsules, respectively), in colloidal drug delivery systems (for example, liposomes, albumin microspheres, microemulsions, nano-particles, and nanocapsules), or in macroemulsions. Such techniques are well known to persons having ordinary skill in the art.

When the active agents are to be used as an injectable material, they can be formulated into a conventional injectable carrier. Suitable carriers include biocompatible and pharmaceutically acceptable phosphate buffered saline solutions, which are particularly isotonic.

For reconstitution of a lyophilized product in accordance with the present disclosure, one may employ a sterile diluent, which may contain materials generally recognized for approximating physiological conditions and/or as required by governmental regulation. In this respect, the sterile diluent may contain a buffering agent to obtain a physiologically acceptable pH, such as sodium chloride, saline, phosphate-buffered saline, and/or other substances which are physiologically acceptable and/or safe for use. In general, the material for intravenous injection in humans should conform to regulations established by the Food and Drug Administration, which are available to those in the field. The pharmaceutical composition may also be in the form of an aqueous solution containing many of the same substances as described above for the reconstitution of a lyophilized product.

The active agents can also be administered as a pharmaceutically acceptable acid- or base-addition salt, formed by reaction with inorganic acids such as hydrochloric acid, hydrobromic acid, perchloric acid, nitric acid, thiocyanic acid, sulfuric acid, and phosphoric acid, and organic acids such as formic acid, acetic acid, propionic acid, glycolic acid, lactic acid, pyruvic acid, oxalic acid, malonic acid, succinic acid, tauric acid, maleic acid, and fumaric acid, or by reaction with an inorganic base such as sodium hydroxide, ammonium hydroxide, potassium hydroxide, and organic bases such as mono-, di-, trialkyl and aryl amines, and substituted ethanolamines.

In certain embodiments, the present disclosure includes an active agent composition wherein at least one of the active agents is coupled (e.g., by covalent bond) directly or indirectly to a carrier molecule.

Various embodiments of the present disclosure will be more readily understood by reference to the following examples and description, which as noted above are included merely for purposes of illustration of certain aspects and embodiments of the present disclosure, and are not intended to be limiting. The following detailed examples and methods which describe various compositions of the present disclosure and are to be construed, as noted above, only as illustrative, and not limitations of the disclosure in any way whatsoever.

### Example 1: SHetA2 - PRIMA-1/PRIMA-1^{MET} Drug Combinations

### Methods

### Cell lines, Plasmids and Drugs

Ovarian cancer cells lines A2780 (RRID:CVCL_0134), OV-90 (RRID:CVCL_3768), SKOV3 (RRID:CVCL_0532), COV362 (RRID:CVCL_2420), OVSAHO (RRID:CVCL_3114), Caov3 (RRID:CVCL_0201), and OVCAR4 (RRID:CVCL_1627) (all from American Type Culture Collection/ATCC Manassas, VA, USA) were cultured in RPMI1640 medium containing 10% FBS and 1X antibiotic/antimycotic. The MESOV/GFP-luc (RRID:CVCL_CZ92) cell line (gift from Dr. Francois Moisan, Stanford University) (MESOV) was cultured in DMEM-high glucose medium with 10% FBS. SKOV3 cells stably transfected with mutant p53 (R248W, R273H) or parent vector were provided by Jeremy Chien, PhD (University of Kansas Medical Center). Human fallopian tube secretory epithelial cells (hFTSECs) were isolated from fallopian tube fimbriae surgical specimens under an IRB approved protocol. All cell lines were authenticated by autosomal short tandem repeat (STR) profiles determination within three years and comparison with reference databases by the University of Arizona Genetics Core and IDEXX BioAnalytics (Columbia, MO, USA). Experiments were performed with cell lines free of mycoplasma contamination. Cultures were transfected with plasmids HSPA9 pLX304 or pLKO-p53-shRNA-427, PG13-Luc (Addgene, Watertown, MA) and treated with SHetA2 and PRIMA-1 (National Cancer Institute PREVENT Program), and PRIMA-1^{MET} (Cayman Chemicals, Michigan, USA) dissolved in dimethyl sulfoxide (DMSO). Caspase 3 inhibitor Z-DEVD-FMK and N-acetyl-L-Cysteine (Cayman Chemicals, Michigan, USA) were used to study mechanism of drug action.

### TP53 Mutation Identification

Exome sequencing data from 429 TCGA ovarian cancer cases was analyzed for genome-wide mutations. The genomic coordinates of these mutations were converted from human genome build hg18 to hg19 using the UCSC Genome Browser liftOver tool (http://genome.ucsc.edu/cgi-bin/hgLiftOver). A total of 405 high-confidence mutations were located within the transcript locus of p53. Mutations were assigned to exons of ENSEMBL p53 transcripts on the basis of their location and predicted mutation effect.

### Mortalin Expression in Ovarian Tissue

The Gynecologic Oncology Group (GOG) Disease Progression Tissue Microarray (TMA) was stained for mortalin using Abcam rabbit polyclonal antibody ab53098. Prior to staining the TMA, antibody dilutions and other immunohistochemical staining conditions were optimized on HGSOC sections using no primary antibody as negative controls. Heat-induced epitope retrieval was performed on formalin-fixed, paraffin-embedded TMA sections using a pressurized decloaking chamber (Biocare Medical, Pacheco, CA, USA) in citrate buffer (pH 6.0) at 99°C for 18 min and then in 3% hydrogen peroxide at room temperature for 20 min. After incubation with primary antibody, positive signal was developed with a rabbit polymer-horseradish peroxidase secondary detection kit (Biocare Medical, Pacheco, CA, USA) and developed diaminobenzidine (Sigma, St. Louis, MO, USA). Digitized images of IHC-stained slides were generated and evaluated for mortalin positive staining intensities using the Positive Pixel Count algorithm software (Aperio Technologies, Inc., Vista, CA, USA). Positivity scores (total number of positive pixels divided by total number of pixels) of the mortalin staining was derived separately for epithelial and stromal cells by tuning a Genie algorithm to differentiate between these two cell types on mortalin-stained ovarian tissues. Marked-up images of the staining intensity measurements generated by the Aperio imaging software were reviewed manually and individual cores that exhibited artifacts, lack of tumor on the section, or inaccurate categorization of stromal versus epithelial cells were eliminated from the analysis. Each patient was represented on the slide by one to three sections. Individual cores that exhibited >10 fold difference from the two other cores from the same patient were eliminated from the averages.

### Subcellular Fraction Enrichment

Cytoplasmic and nuclear protein fractions were isolated from cultures using NE-PER Nuclear and Cytoplasmic Extraction Reagents (ThermoFisher, Waltham, MA, USA) and the Mitochondria/Cytosol Fractionation Kit (ThermoFisher, Waltham, MA, USA) and protein concentrations determined with BCA reagent (ThermoFisher, Waltham, MA, USA).

### Western Blots

Proteins were extracted from cell cultures using M-PER mammalian protein extraction reagent (ThermoFisher, Waltham, MA, USA) and volumes corresponding to 30 µg determined with BCA reagent (ThermoFisher, Waltham, MA, USA) were electrophoresed into a SDS-PAGE gel and transferred to PVDF membranes. Membranes were blocked (5% dry skim milk powder in TBST buffer) for at least 1h or overnight, washed thrice with TBST and then incubated with primary antibody: p53 DO-1, β-actin, histone H3, GAPDH (Santa Cruz Biotechnology, Dallas, TX, USA) and mortalin, Tom20, V5 tag antibodies, p21, PARP, SLC7A11, caspase 3 (Cell Signaling Technology, Danvers, MA, USA) for at least 1h or overnight. After three washes, membranes were incubated with anti-mouse HRP conjugated (Santa Cruz Biotechnology, Dallas, TX, USA) or anti-Rabbit HRP conjugated (Cell Signaling Technology, Danvers, MA, USA) for 40 min. Membranes then were washed and developed with ECL reagents (BioRad, Hercules, CA, USA). Bands were imaged and quantified (ChemiDoc imaging system with Image Lab Software/BioRad).

### Immunofluorescence

Chamber slides (Lab-Tek II) were seeded with 1 × 10⁴ cells in 1 ml medium and incubated up to 50-70% confluence. Cells treated with SHetA2 or an equivalent volume of DMSO were fixed in 2% paraformaldehyde in PBS, pH 7.4 for 30 min. at room temperature, washed with PBS three times and then permeabilized with 0.5% Triton X-100 in PBS, 7.4 for 5 minutes. After three PBS washes, cells were incubated in PBS containing 0.1% BSA in PBS for 30 min. at 37°C and then with p53 DO-1 antibody for 1h at 37°C. After three PBS washes, cells were incubated with the secondary antibody tagged with fluorochrome (Alexa fluor 488 or 647) for 1h at 37°C and washed thrice in PBS. Nuclei were stained with 300 nM DAPI for 7 min at room temperature and cells were washed thrice in PBS. Cells were covered in Prolong Gold mounting medium (Life Technology, Carlsbad, CA, USA) and 1 mm thick cover glasses and left to dry overnight at room temperature and then imaged with a confocal microscope Leica SP2 (Leica, Wetzlar, Germany) at 63X.

### MTT Cytotoxicity Assay

Cells plated at 2000 cells/well in 96 well microtiter plates were incubated for 36-48 h and treated with a range of doses and combinations of SHetA2 and PRIMA-1 or PRIMA-1^{MET}, or the same volume of DMSO solvent for 72 h of incubation. The percentage of growth for single drug treatments or fold effect (1 - ratio of treated/untreated growth) was measured using the Cell Titer 96 AQueous Non-Radioactive Cell Proliferation Assay (Promega, Madison, WI, USA) and the BioTeK SYNERGY H1 (BioTeK, Winooski, VT, USA). Each experiment was performed in triplicate and repeated 2-3 times.

### Transfection of Cells

1×10⁶ cells were electroporated with plasmid (5-10 µg) using the Neon transfection unit (Life technology, Waltham, MA) at 1170 V, 30 width and 2 pulses. Transfected cells were seeded onto 6-well and 96-well plates, and incubated for 36-48h prior to evaluation by Western blot and MTT assays, respectively. A stable cell line of A2780 cells transfected with pLKO-p53-shRNA-427 was generated by transfecting A2780 cells using the electroporation conditions above. Transfected cells were titrated for the optimum puromycin concentration needed for the selection, which was determined to be 20 µg. Transfected cells were grown under puromycin pressure for 2 weeks and after that continued to grow in growth medium without puromycin. The puromycin-resistant cell line was evaluated for p53 knockdown using Western blot.

### p53 Luciferase Reporter Assay

To determine the transcriptional activity of p53, MESOV cells were transfected with PG13-Luc plasmids. Approximately 5,000 transfected cells were seeded on to each well of 96-well plate and incubated overnight. These cells were treated with either control solvent, 5 µM SHetA2 or 60 µM PRIMA-1^{MET} for 24 h. Luciferase activity of the reporter was measured with the One-glo luciferase reporter assay kit (Promega, Madison, WI, USA) and a Biotek Synergy H1 plate reader (BioteK, Winooski, VT, USA). The MTT assay was performed in parallel cultures and used to normalize the p53 reporter activity to the amount of viable cells.

### Drug Interactions

The potencies (IC₅₀'s) of SHetA2 or PRIMA-1 were determined using GraphPad Prism 6 software analysis of average MTT Optical Density vs drug dose. The drugs were mixed at a 1:1 ratio of their IC₅₀ concentrations and a series of 2-fold dilutions evaluated in the MTT assay. Single drug treatments at doses surrounding the IC₅₀ values were evaluated parallel. All treatments were performed in triplicate and averages used to draw isobolograms and calculate combination indices (CI) and dose-reduction indices (DRIs) using CompuSyn Software according to the Chou and Talalay method (op cit). All experiments were repeated at least twice for each cell line to confirm results.

### Quantitative reverse transcriptase polymerase chain reaction (qRT-PCR)

SKOV3 parental and SKOV3 R273H p53 mutant cells were grown to 80% confluency in a 100 mm tissue culture dish and then treated with SHetA2 10 µM or PRIMA-1 20 µM or both drugs combined for 16h after which cells were harvested and RNA was isolated using RNeasy Mini Kit (QIAGEN). RNA quality and concentration was evaluated by measuring the OD260/280 ratio. The cDNA was synthesized from 1 µg RNA using RT2 First Strand Kit (Qiagen, Hilden, Germany) and then Real-time PCR was performed by using RT2 SYBR Green master mix (GeneCopoeia, Rockville, MD, USA) and the BioRad CFX96 Real Time PCR detection system (Bio Rad, Winooski, VT, USA). The real time expression values were analyzed by calculating ΔΔCT and mean fold change (2^- (ΔΔCT)) values.

### Assays for Reactive Oxygen Species (ROS) and ATP

The ROS-Glo H₂O₂ Assay and CellTiter-Glo 2.0 Assay (Promega, Madison, WI, USA) kits were used to measure ROS and ATP, respectively, in cells cultured on 96-well plates with various combinations of SHetA2 (5 µM, 10 µM) and PRIMA-1 or PRIMA-1^{MET} (25 µM) combinations, and the luminescence endpoints were measured with a BioTeK SYNERGY H1. The ROS assay luminescence endpoints were normalized to total viable cells using the MTT assay. For ROS to ATP ratio determination, the ROS luminescence endpoint was normalized to total protein content (µg/µl) measured with the BCA reagent in parallel wells, and the ATP concentrations were derived from a standard curve and normalized to total protein content. All assays were performed in triplicate and repeated three times.

### Animal Model

Four-week-old female athymic nude mice (NCr-Foxn1^{nu}) were obtained from Taconic Biosciences under an approved Institutional Animal Care and Use Committee (IACUC) protocol. Mice were acclimatized for two weeks, fed with a standard diet and ear tagged during the acclimatization period. The timing to tumor development after intraperitoneal injection with various amounts of MESOV cells was determined in a pilot study prior. In the intervention experiment, 6-week-old mice were injected with 20 million MESOV human ovarian cancer cells in their intraperitoneal region. Drug treatment was initiated 4 weeks after the injection of cancer cells. Mice were randomized to four treatment groups before the start of treatment based on their body weight. There was no significant (one-way ANOVA, p >0.999) difference in body weight between groups at the start of treatment. Twelve mice were allocated to each group based on the power analysis performed while designing experiment. The sample size was determined to provide over 80% power to detect a 915 mm³ difference in tumor volume between PRIMA-1^{MET} treatment alone and the drug combination treatment using a two-sided .05 level t-test. SHetA2 was administered by gavage at 60mg/kg in 30% Kolliphor HS15 (Sigma). Controls were gavaged with Kolliphor HS15 (30% in water). PRIMA-1^{MET} (10mg/kg) was dissolved in phosphate buffered saline and injected into the peritoneum of mice every other day. Initially SHetA2 dosing was administered every day for two weeks in SHetA2 alone and drug combination groups, and then switched to every other day for the next three weeks due to the stress caused to the mice by daily gavage. Oral gavaging was performed with sterile disposable plastic feeding tubes 20ga x 38mm (Instech Laboratories, Inc., Plymouth Meeting, PA, USA). Three mice in the drug combination group died after two weeks of drug treatment due to gavaging incidents. Body weights were recorded and animal health was monitored weekly. After the end of drug intervention period, all mice were euthanized by CO₂ asphyxiation followed by exsanguination, their tumors and other vital organs were collected for further analysis. After measuring the total tumor weight, a portion of the tumors were snap frozen in liquid nitrogen and another portion was formalin fixed. The formalin fixed tissue samples were paraffin embedded, sectioned and stained with hematoxylin and eosins for histology studies. An experienced pathologist reviewed blinded histology slides for kidney and liver toxicity.

### Statistics

For tissue culture studies, the normality of data and statistical significance were analyzed using Prism 8 Software (GraphPad, San Diego, CA, USA). Comparison of drug effects in the presence and absence of p53 knockdown or mortalin overexpression were done using T-tests corrected for multiple comparisons using the Holm-Sidak method. Comparison of drug combinations compared to single drug treatments were done using a two-way ANOVA with a Tukey's multiple comparison test. P values < 0.05 were considered significant.

Presence or absence of tumor at the end of the treatment period was used as the primary outcome measure. A binary variable (no tumor) was created which equaled 1 if the tumor weight was zero and equaled 0 otherwise. Two by two logistic regression was used to compare the four treatment groups (Untreated control, SHetA2, PRIMA1^{MET}, SHetA2+PRIMA1^{MET} combination) in the study. Mice in the first 3 groups had complete data, while 3 mice in the combo group died early and had zero tumor weights. To assess the synergistic effects of SHetA2 and PRIMA1^{MET}, a logistic regression model was used to analyze the no tumor variable with two factors SHetA2 (indicating whether SHetA2 was part of the treatment) and PRIMA1^{MET} (indicating whether PRIMA1^{MET} was part of the treatment) and their interaction. The interaction coefficient was not included in the model because it was not significant, which resulted in an additive model. Statistical Analysis (SAS 9.4) was used to perform the analysis. All of the tumors found and excised during necropsy were weighed simultaneously to obtain the tumor burden, which was considered a continuous variable. Tumor burdens between the different treatment groups were compared by the Kruskal-Wallis test using Prism 8.0 (GraphPad Software, San Diego, CA, USA).

### Results

### Alterations of p53 and Mortalin in Ovarian Cancer Specimens

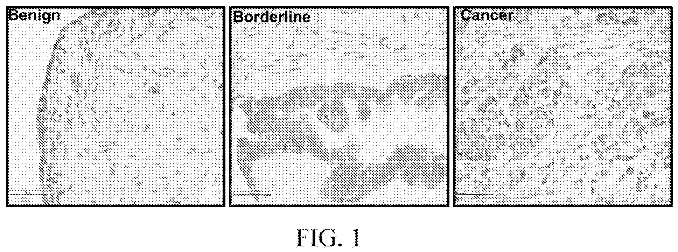

Various missense p53 mutations commonly present in cancer exert different functions that could cause dissimilar consequences on responses of tumors to therapy, and therefore have potential to predict treatment response when evaluated individually. To evaluate the profile of TP53 mutations in TCGA HGSOC data, Ensembl exons of p53 genomic regions corresponding to various domains of the p53 protein were evaluated individually (See FIG. 1A of Provisional Application Serial No. 62/730,345, filed Sept. 12, 2018). TP53 exon mutations in TCGA HGSOC data occurred at a 94.4% (405/429) frequency. The majority (58%, 234/405) were missense mutations within the DNA binding domain (aa 102-292). Another 3% (12/405) were in regions outside of the DNA binding domain. The types of mutations known, or likely, to result in loss of full-length p53 protein expression from one allele (frameshift deletions and insertions, nonsense mutations and splice site mutations) totaled 39% (157/405). The most frequently mutated amino acids in p53 protein were R248, R273, R175, G245, R249 and R282. HSPA9 (mortalin) gene mutations were infrequent.

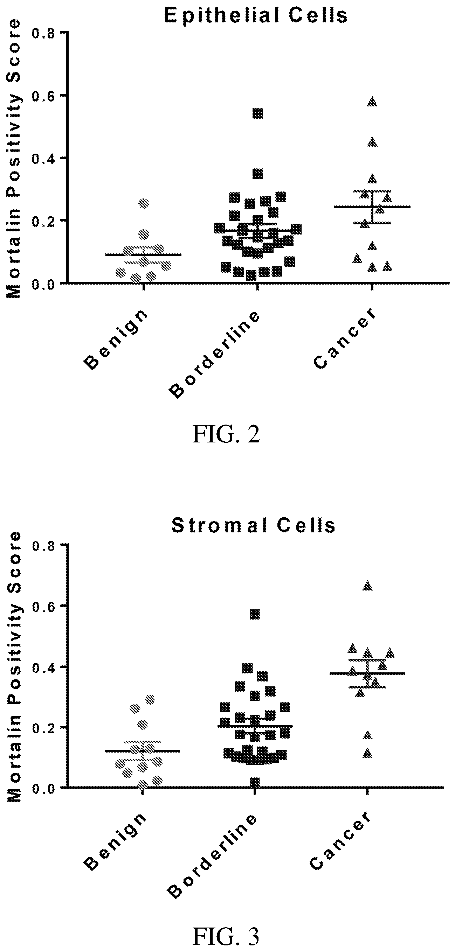

The status of the SHetA2 drug target mortalin in a patient's tumor is also likely to affect treatment outcome. The present analysis of TCGA data found no association of mortalin mutations with ovarian cancer. IHC analysis of mortalin expression in a series of serous benign, borderline and cancerous tissues on a TMA demonstrated punctate expression of mortalin in both epithelial and cancer cells consistent with its primary localization in mitochondria (FIG. 1). Mortalin positivity scores in epithelial and stromal cells significantly increased in a stepwise manner from benign to borderline to cancerous tissues (FIGS. 2 and 3, respectively; Kruskal-Wallis test p = 0.0282 for epithelial cells; p = 0.0002 for stromal cells).

### Effects of SHetA2 on p53 Cellular Localization

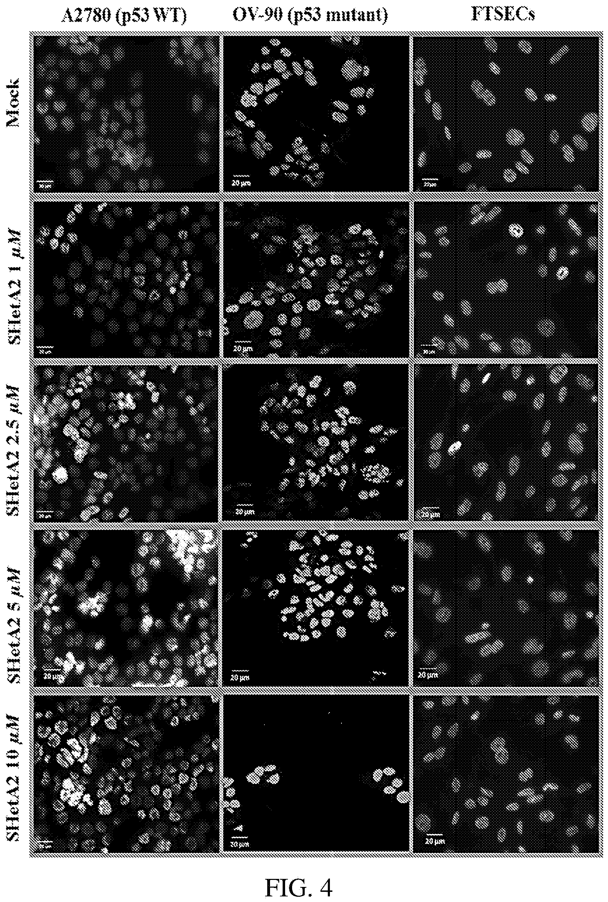

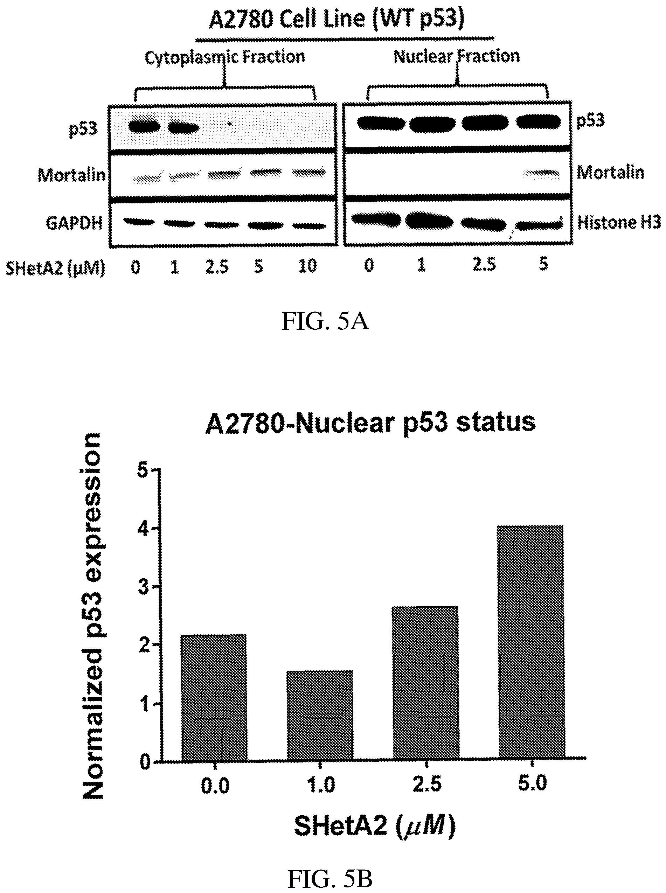

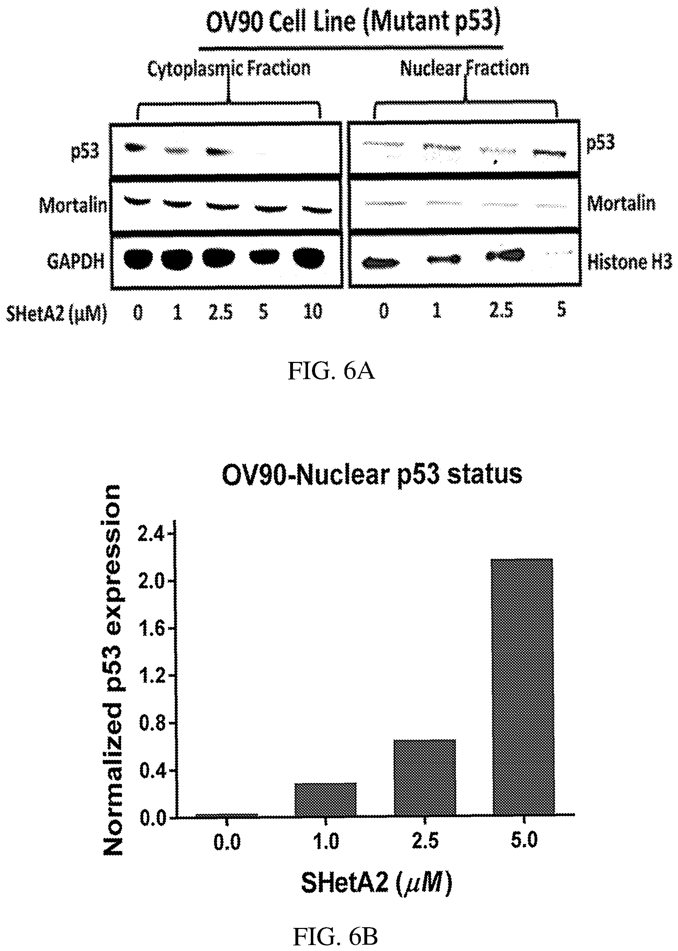

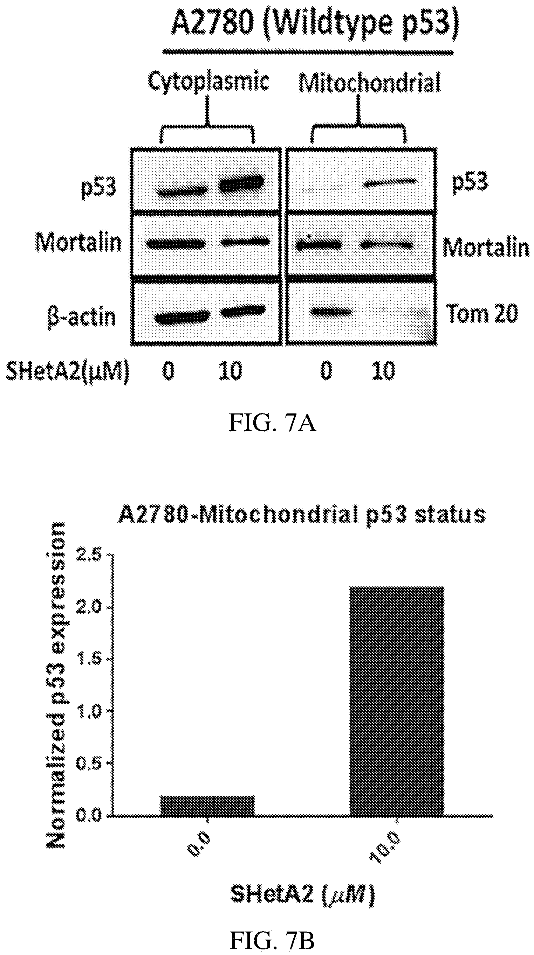

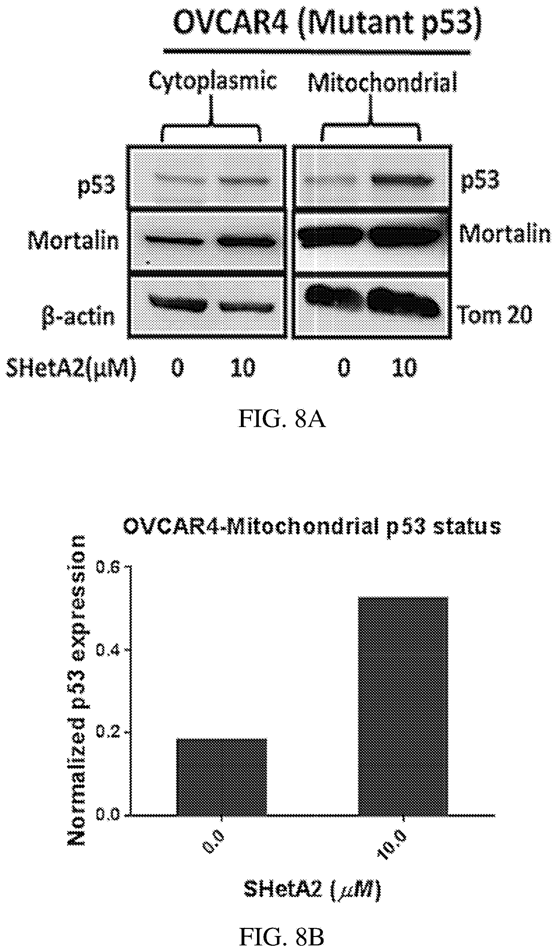

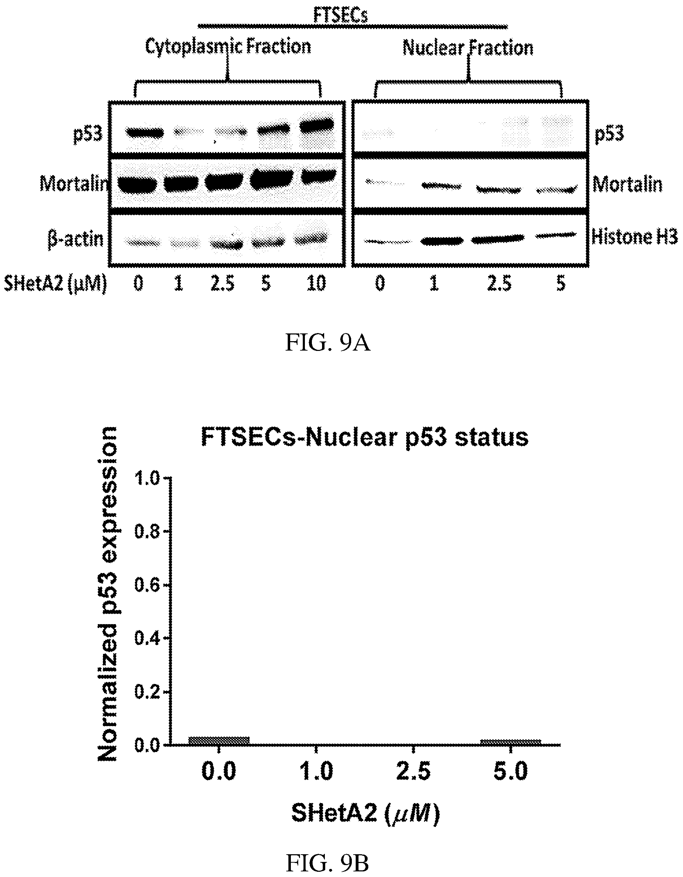

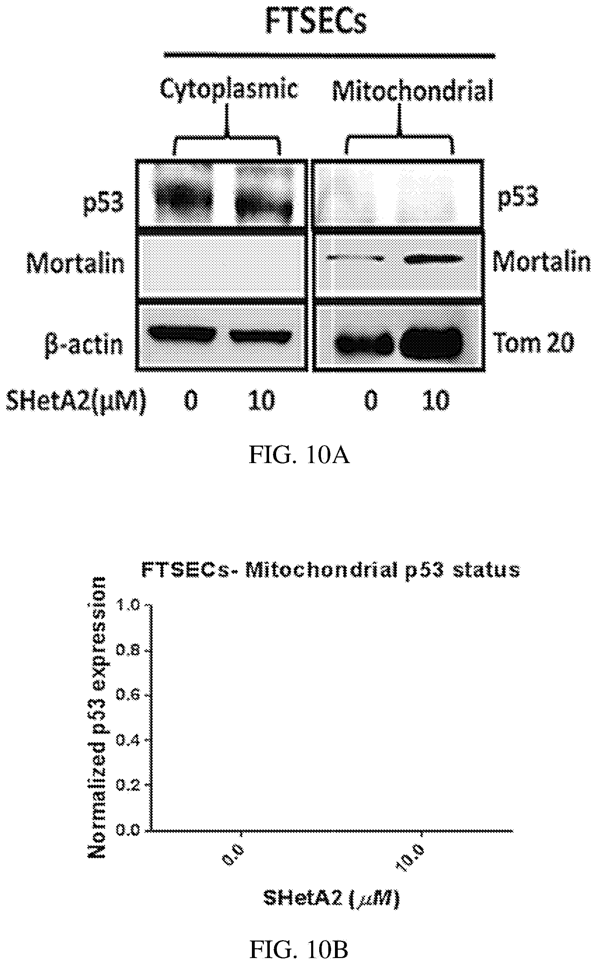

We predicted that SHetA2 disruption of mortalin/p53 complexes would cause p53 to translocate from the cytoplasm where it is sequestered by mortalin to accumulate in its nuclear and mitochondrial sites of action. Immunofluorescent imaging confirmed SHetA2 dose-dependent elevation of both wild type and mutant p53 in nuclei of ovarian cancer cell lines, but not in hFTSECs (FIG. 4). Very little p53 was detectable in the cytoplasm likely due to masking of the epitope by binding proteins and the short p53 half-life. Western blots demonstrated SHetA2 dose-dependent decreases in cytoplasmic fractions and increases in nuclear (FIGS. 5-6) and mitochondrial (FIGS. 7-8) fractions of the A2780 wild type p53 and OV90 S215R or OVCAR4 m-p53 cell lines. SHetA2 caused minimal effects on p53 cellular localization in human fallopian tube secretory epithelial cells (hFTSECs) (FIGS. 9-10). In contrast to p53, mortalin cellular or nuclear levels were not affected by SHetA2 treatment in these cell types except for one instance of nuclear accumulation in A2780 cells treated with 5 µM SHetA2 (FIGS. 5,6,9).

### Effects of SHetA2 and PRIMA-1 as Single Agents

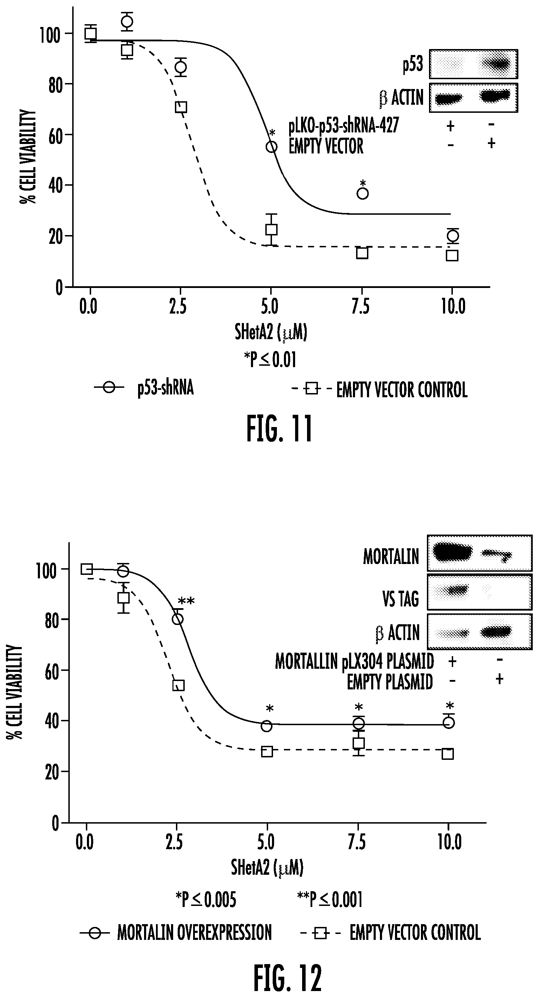

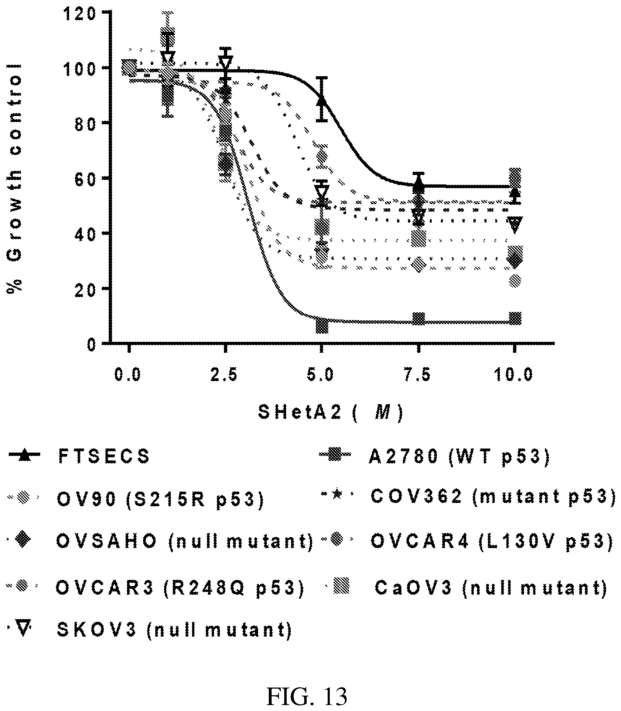

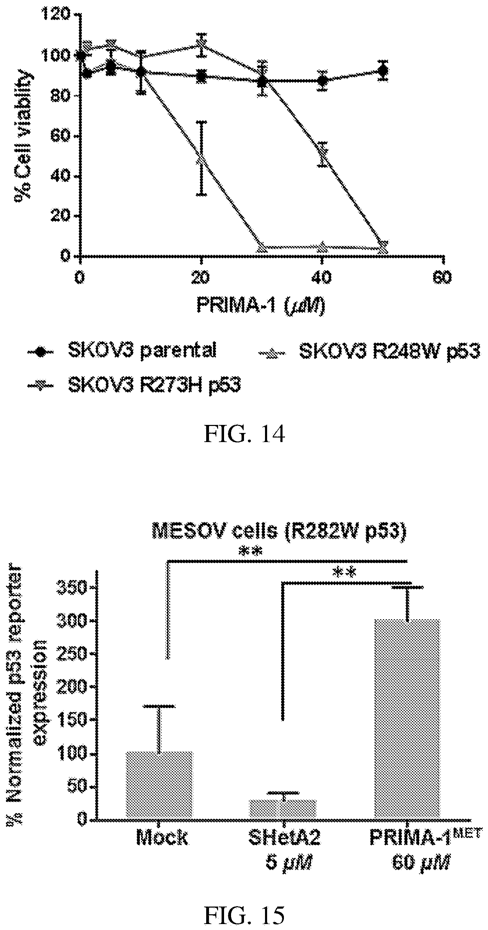

shRNA knockdown of p53 reduced SHetA2 potency in A2780 cells from a 2.8 µM to 4.8 µM IC₅₀ (FIG. 11). Overexpression of mortalin in A2780 cells also reduced SHetA2-induced cytotoxicity (FIG. 12), likely due to mortalin sequestering SHetA2 away from mortalin/p53 complexes. Ovarian cancer cell lines expressing m-p53 were more resistant to SHetA2 in comparison to the wild type p53 A2780 cell line, while hFTSECs were the least sensitive (FIG. 13). SKOV3 p53 null cells permanently transfected to express m-p53 exhibited increased PRIMA-1 sensitivity in comparison to PRIMA-1-resistant parental SKOV-3 cells to different extents depending on the mutant (R248W: 19.98 µM IC₅₀; R273H:IC₅₀ 39.96 µM IC₅₀) (FIG. 14). PRIMA-1^{MET} reactivation of R282W m-p53 in MESOV was observed using a p53 reporter plasmid (FIG. 15).

### Effects of SHetA2 and PRIMA-1/PRIMA-1^{MET} in combination

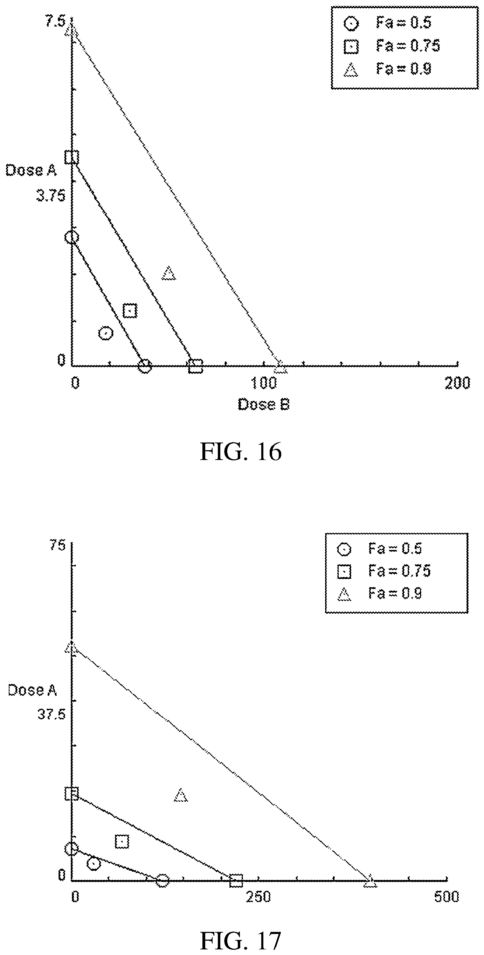

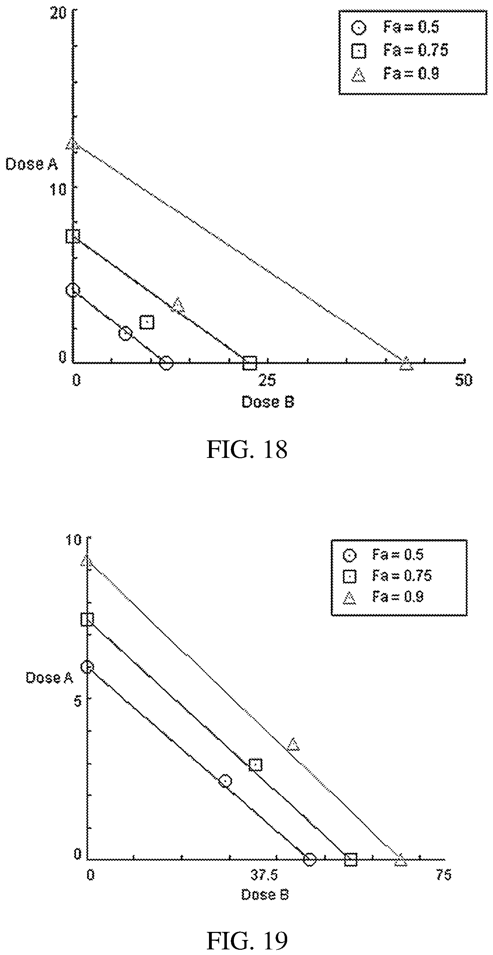

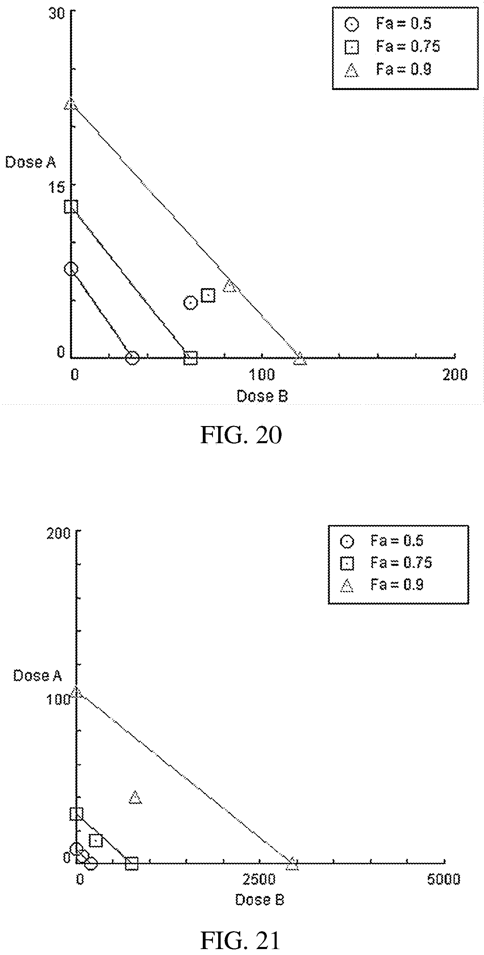

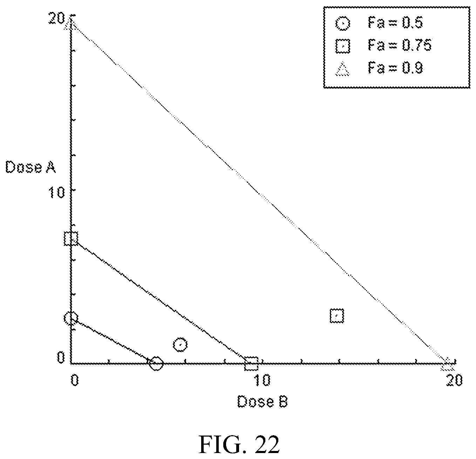

SHetA2 and PRIMA-1 showed synergism in ovarian cancer cells harboring wild type p53 or m-p53, additive interaction in Caov3 p53 null cells and antagonism in hFTSECs (FIGS. 16-20 and Table 1). SHetA2 and PRIMA-1^{MET} also showed synergism in m-p53 MESOV cells and antagonism in hFTSECs (FIGS. 21-22 and Table 1). DRIs of SHetA2 and PRIMA-1/PRIMA-1^{MET} combinations indicated that the combination allows lower doses of each drug to be used without reducing activity (Table 2). For example, for the cell line A2780, in the case of when SHetA2 and PRIMA-1 are used in combination, 3.84 times less SHetA2, and 2.12 times less PRIMA-1 can be used to achieve the same IC₅₀ as that obtained by using either drug alone.

**Table 1. Combination index values at various Effective Doses (EDs) as calculated by CompuSyn using the Chou-Talalay method.**

| **Combinations** | **Combination index (CI)* at** | | | | **Combination analysis result** |
|---|---|---|---|---|---|
| **SHetA2+ PRIMA-1** | **ED₅₀** | **ED₇₅** | **ED₉₀** | **ED₉₅** | |
| A2780 (WT p53) | 0.73 | 0.74 | 0.74 | 0.75 | Synergism |
| MESOV (R282Wp53) | 0.81 | 0.75 | 0.73 | 0.73 | Synergism |
| SKOV3 R248W p53 | 0.96 | 0.75 | 0.59 | 0.50 | Synergism |
| CaOV3 (Null p53) | 1.04 | 1.03 | 1.04 | 1.05 | Additive |
| FTSECs | 2.5 | 1.57 | 0.97 | 0.71 | Antagonism |

| **SHetA2 + PRIMA-1^{MET}** | | | | | |
|---|---|---|---|---|---|
| MESOV (R282Wp53) | 1.01 | 0.82 | 0.66 | 0.57 | Synergism |
| FTSECs | 1.70 | 1.86 | 2.05 | 2.21 | Antagonism |

| | | | | | |
|---|---|---|---|---|---|
| *CI<1, =1 and > 1 indicates synergism, additive effect, and antagonism, respectively. | | | | | |

**Table 2. Dose reduction indices of SHetA2 and PRIMA-1/PRIMA-1^{MET} drug combinations in various cell lines.**

| **Drug Combinations/ Cell lines** | **Dose-reduction index (DRI)* (SHetA2/PRIMA-1 or SHetA2/PRIMA-1^{MET}) at** | | | |
|---|---|---|---|---|
| **SHetA2+ PRIMA-1** | **IC₅₀** | **IC₇₅** | **IC₉₀** | **IC₉₅** |
| A2780 (WT p53) | 3.84/2.12 | 3.72/2.13 | 3.6/2.14 | 3.53/2.15 |
| MESOV (R282Wp53) | 1.79/3.94 | 2.21/3.27 | 2.73/2.71 | 3.15/2.38 |
| SKOV3 R248W p53 | 2.48/1.79 | 3.03/2.38 | 3.70/3.15 | 4.24/3.81 |
| CaOV3 (Null p53) | 2.48/1.56 | 2.74/1.48 | 3.01/1.40 | 3.22/1.35 |
| FTSECs | 1.62/0.52 | 2.37/0.86 | 3.48/1.44 | 4.50/2.03 |

| **SHetA2 + PRIMA-1^{MET}** | | | | |
|---|---|---|---|---|
| MESOV (R282Wp53) | 1.84/2.10 | 2.17/2.76 | 2.56/3.64 | 2.87/4.38 |
| FTSECs | 2.30/0.78 | 2.59/0.67 | 2.90/0.58 | 3.14/0.52 |

| | | | | |
|---|---|---|---|---|
| *Folds of dose reduction when compared with amount of each drug alone. | | | | |

### Evaluation of SHetA2 and PRIMA-1 Synergy Mechanism

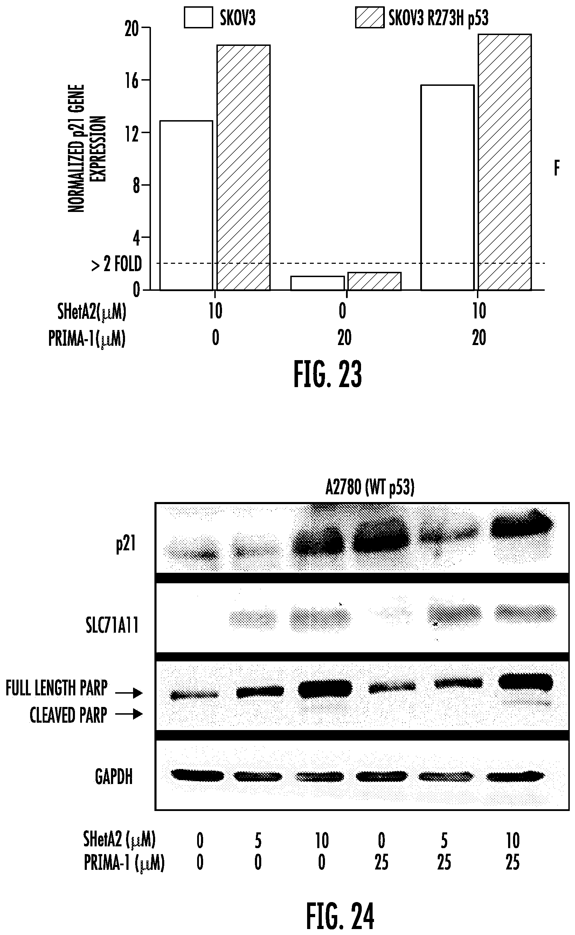

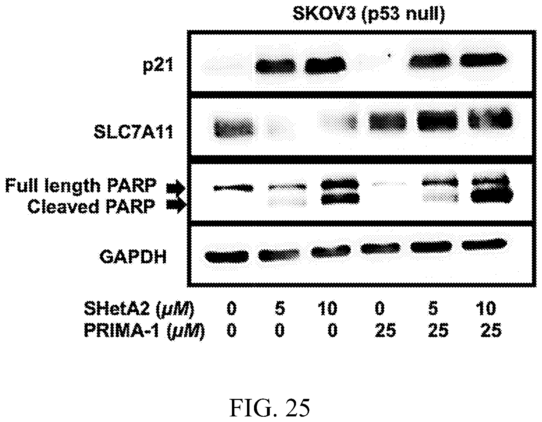

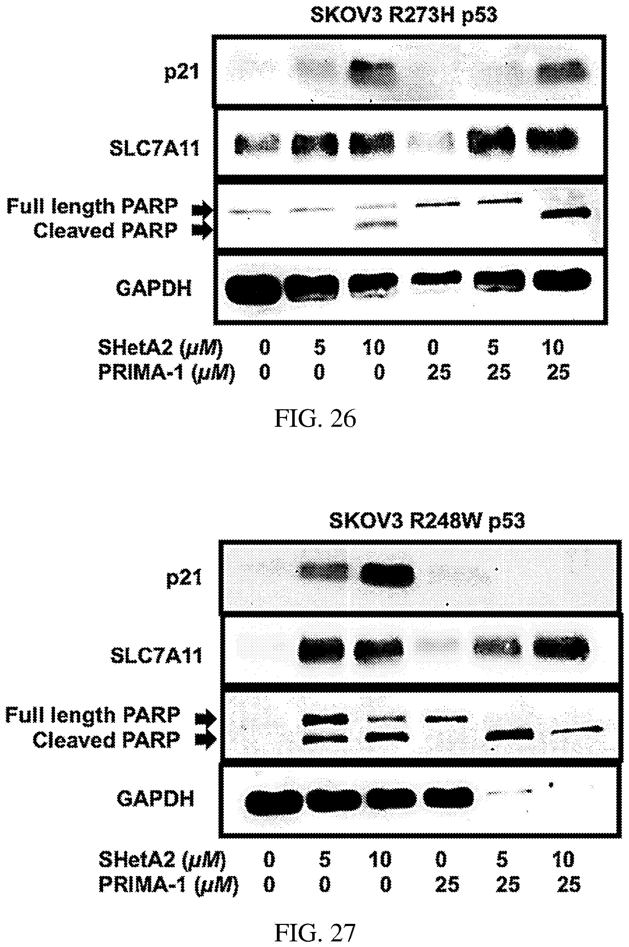

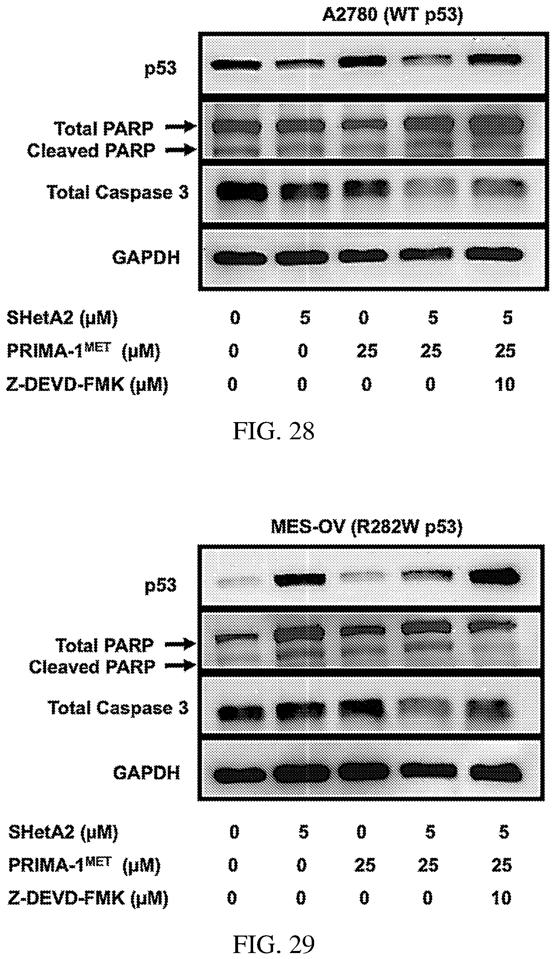

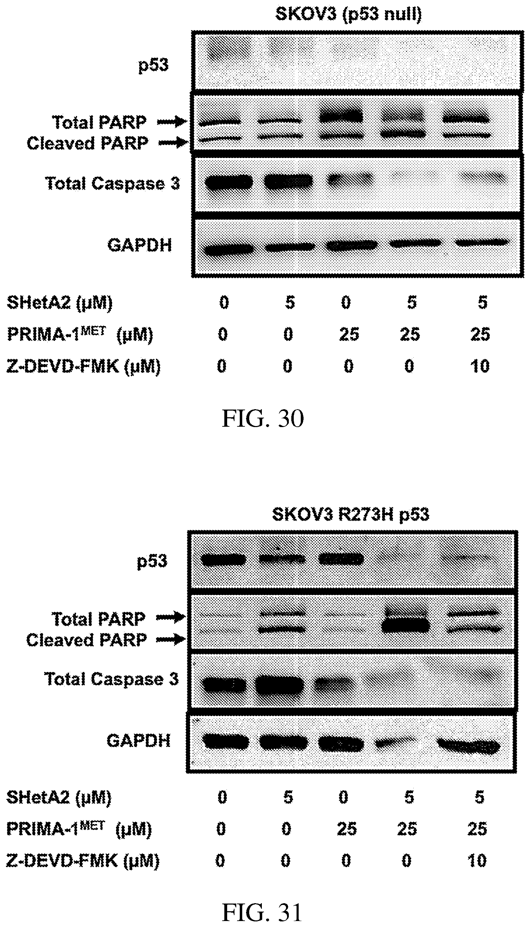

The TP21 gene was evaluated as a marker of p53 transcriptional activity. SHetA2 induced TP21 transcription in p53 null and m-p53 -expressing SKOV3 cells (FIG. 23), and increased p21 protein in all cell lines evaluated regardless of p53 status (FIGS. 24-27). PRIMA-1 did not induce TP21 transcription in m-p53-expressing SKOV3 cells (FIG. 23) and only induced p21 protein expression in A2780 (wt p53) (FIG. 24). PRIMA-1 prevented SHetA2 induction of p21 only in the presence of R248W m-p53, but did not prevent PARP-1 cleavage as an indicator of apoptosis (FIGS. 28-31).

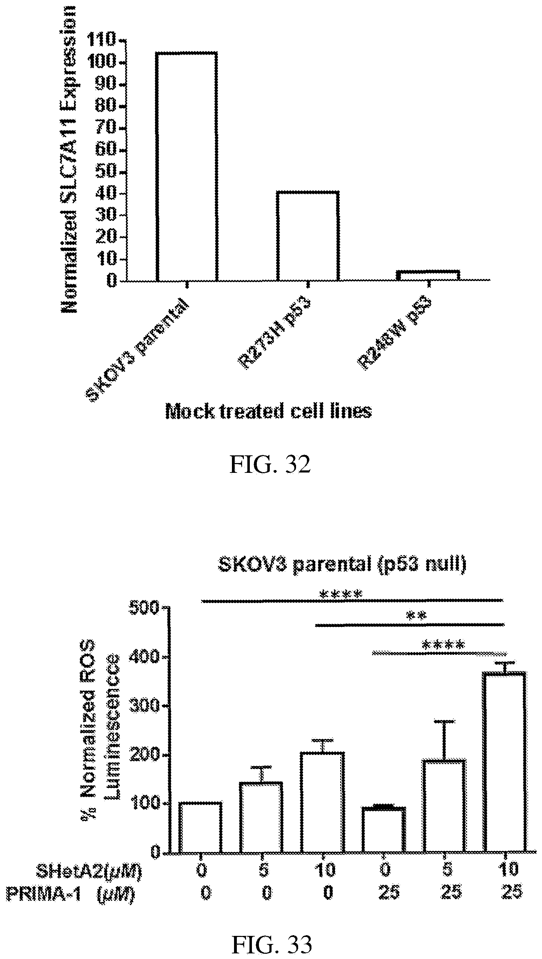

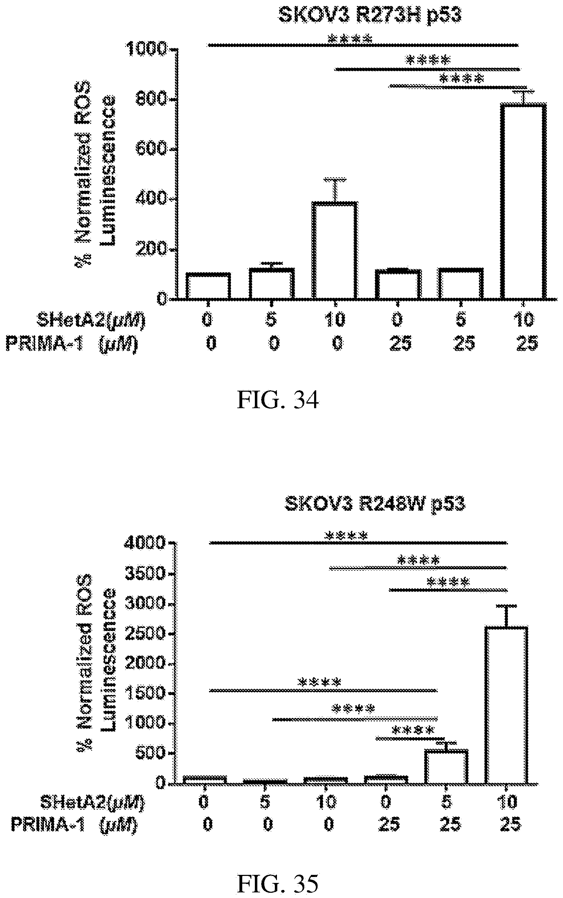

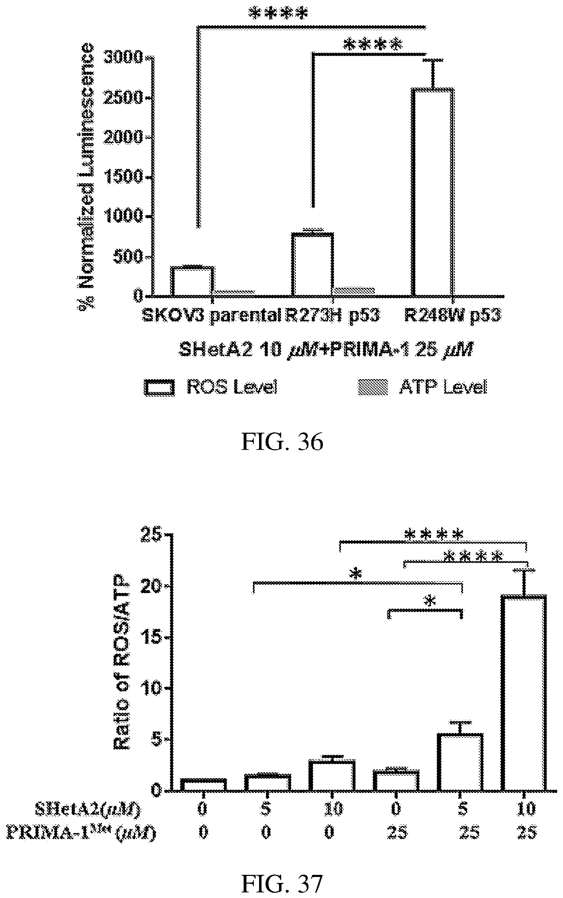

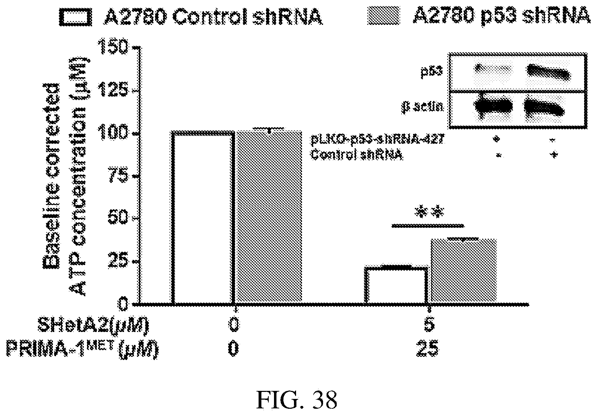

SHetA2 also induced SLC7A11, a cysteine/glutamate antiporter shown to be inversely associated with m-p53 expression and PRIMA-1^{MET} sensitivity by others and confirmed in this study (compare FIGS. 14 and 24-32). The extents of SLC7A11 repression and ROS generation were associated with PRIMA-1 sensitivity (compare FIGS. 14 and 33-36). Involvement of ROS in the synergy mechanism was suggested by higher levels of ROS generation in SKOV3 cells treated with SHetA2 and PRIMA-1 in comparison to single drug treatments, which occurred in an additive manner in p53 null cells and in a synergistic manner in m-p53 expressing cells (FIGS. 33-35). Levels of cellular ATP were decreased as levels of ROS were increased in SKOV3 p-53 null and m-p53 cells treated with SHetA2 and PRIMA-1 (compare FIGS. 14 and 36). ATP levels were also reduced in association with increased ROS by combined treatment with SHetA2 and PRIMA-1^{MET} in A2780 wild type p53 cells, and this effect was antagonized by p53 shRNA (FIGS. 37-38). Involvement of apoptosis in the synergy mechanism was verified by caspase 3-inhibitor, Z-DEVD-FMK, reduction of caspase 3 and PARP-1 cleavage across all cell lines treated with SHetA2 and PRIMA-1^{MET} (FIGS. 28-31).

### Evaluation of SHetA2 and PRIMA-1^{MET} in a Model of Secondary Chemoprevention

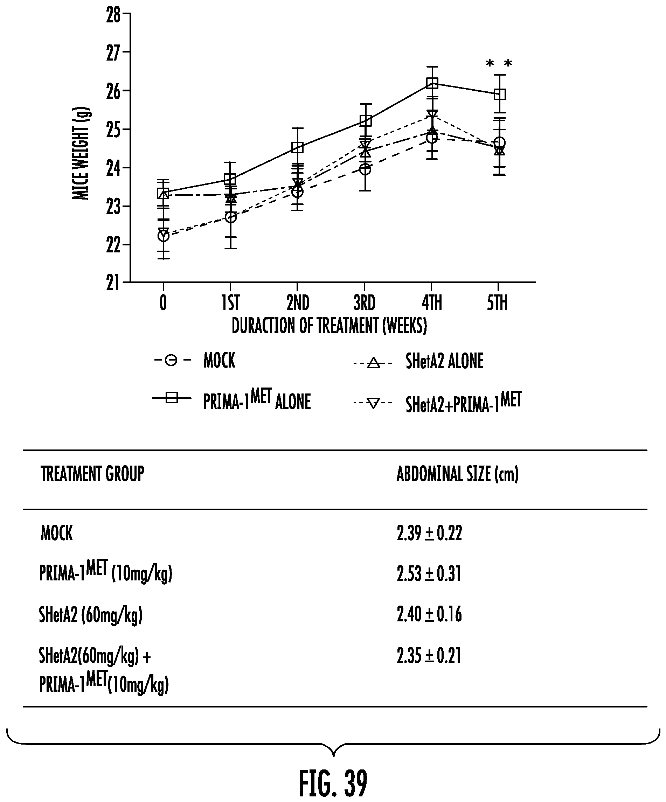

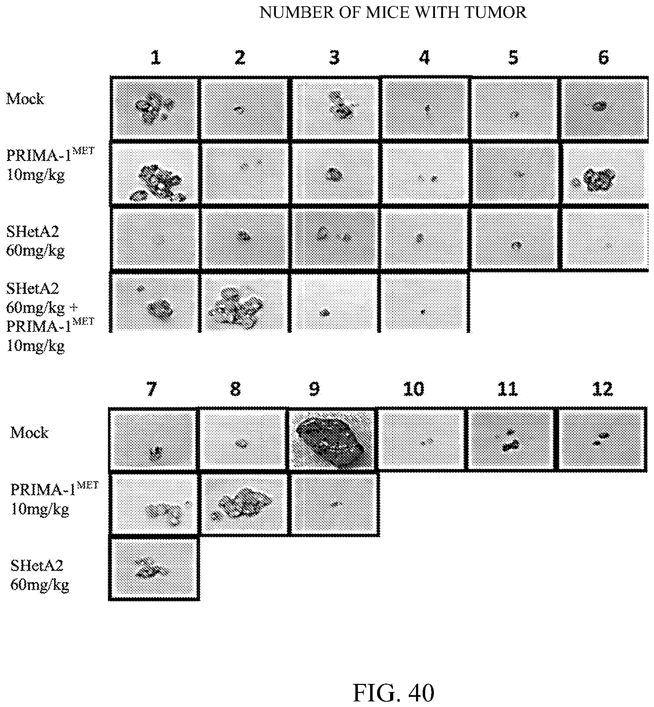

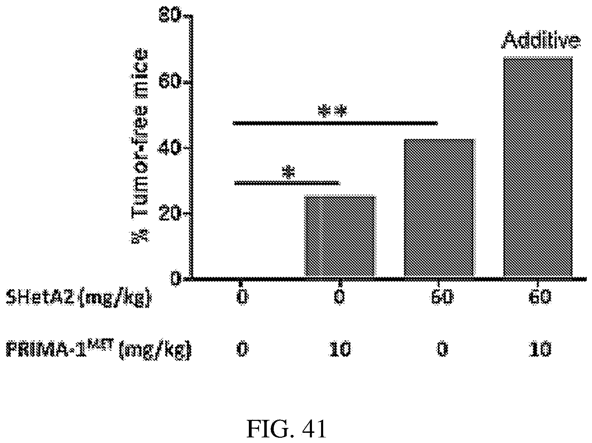

To model maintenance therapy for ovarian cancer, effects of SHetA2 and PRIMA-1^{MET} on establishment of tumors from MESOV cells injected into the peritoneum of immunocompromised mice were evaluated. Treatment was initiated 28 days post-injection and continued for 38 days. Treated mice appeared normal, however the average body weights of the PRIMA-1^{MET} group were significantly higher than the mock group (FIG. 39). This was attributed to reduced gavage-associated stress and development of ascites in the PRIMA-1^{MET} group (FIG. 39). At necropsy, all control group mice had solid tumor nodules with variable numbers and sizes (FIG. 40). Tumors were commonly observed on uterine horns, intestine, chest cavity, stomach, near the spleen and below the kidney and pancreas. Tumor-free rates were 0% in the untreated controls, 25% in the PRIMA1^{MET} treatment group, 42% in the SHetA2 treatment group, and 67% in the combination treatment group (FIG. 41). Logistic regression indicated that the interaction between SHetA2 and PRIMA1^{MET} was not significantly synergistic (p=0.973). A subsequent additive model showed that both SHetA2 (p=.004, OR=10.384, 95% CI: 2.158, 48.965) and PRIMA1^{MET} (p=0.048, OR=4.464, 95% CI: 1.014, 19.655) significantly prevented tumor development, and that these two drugs acted additively in preventing tumor establishment.

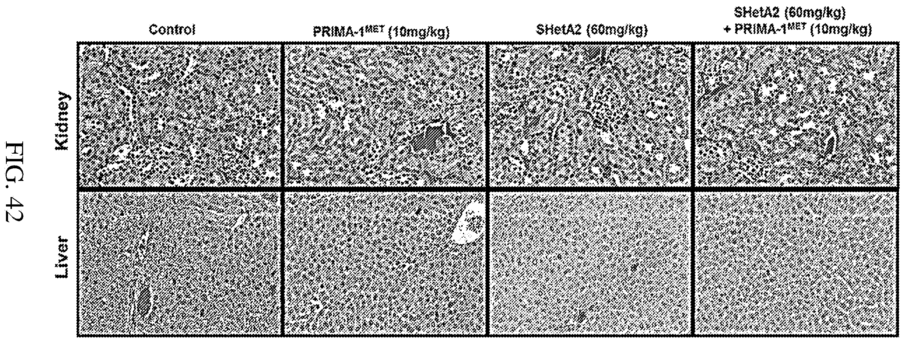

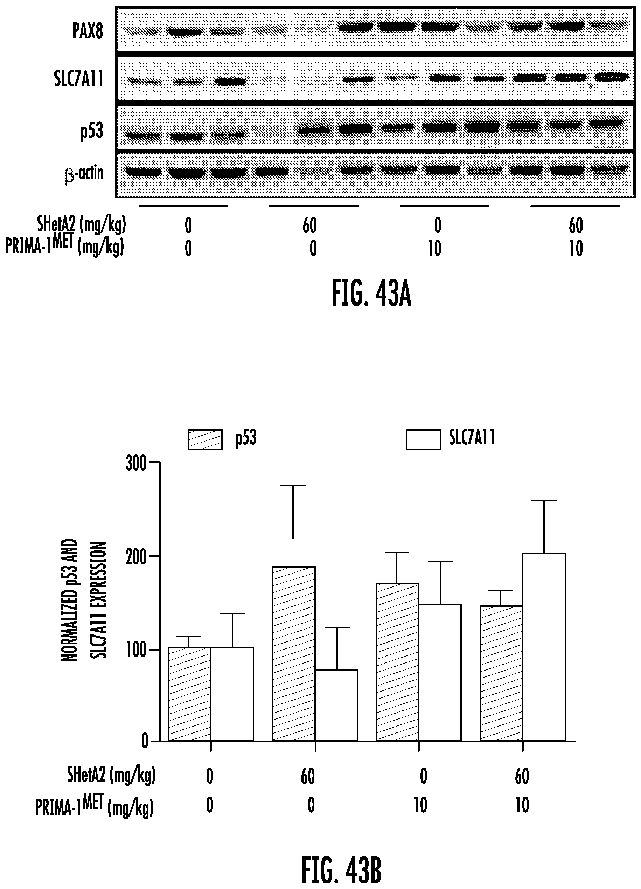

Histologic analysis of kidney and liver collected upon necropsy showed no evidence of toxicity (FIG. 42). Western blot analysis of tumors confirmed expression of Pax8, a marker of high-grade serous ovarian cancer (FIG. 43A). There was no association of p53 expression with treatment (FIG. 43A). SLC7A11 was increased significantly in the drug combination group compared to control group (FIG. 43B).

These results provide justification for targeting m-p53 and mortalin in development of ovarian cancer therapy. TCGA data analysis revealed a 58% m-p53 rate in HGSOC. TMA analysis demonstrated elevated mortalin expression in serous cancer. Two drugs, SHetA2 and PRIMA-1^{MET}, which wield complementary mechanisms targeted at m-p53 and mortalin, exerted synergistic cytotoxicity against ovarian cancer cell lines expressing m-p53 and additive activity in a p53 null cell line. The antagonism observed in hFTSEC cells suggests that this combination will not be toxic in clinical trials. The significant in vivo prevention of tumor establishment by each drug alone and the additive activity when used in combination validate the tissue culture results.

We predicted that SHetA2 would increase p53 activity because it releases p53 from binding to mortalin, and mortalin can sequester p53 in the cytoplasm away from its apoptosis sites of action in stressed cancer cells. In the present work, SHetA2 caused p53 accumulation in nuclei and mitochondria in ovarian cancer cells, but not in hFTSECs. This differential effect may explain the lack of observed toxicity for SHetA2. Targeting mortalin/p53 has limitations in HGSOC, due to high m-p53 frequency and our observation that cell lines expressing endogenous or exogenous m-p53 exhibited increased SHetA2 resistance compared to wild type or p53 null cell lines.

We hypothesized that a combination of a heteroarotinoid such as SHetA2 with a p53 reactivator drug would be synergistic in m-p53 cell lines, because SHetA2 would increase the amount of p53 available to induce apoptosis while the p53-reactivator would restore apoptosis function to released m-p53. The results support this hypothesis in that SHetA2 and PRIMA-1 or PRIMA-1^{MET} acted synergistically in cytotoxicity assays of ovarian cancer cell lines expressing m-p53, but only additively in p53 null lines, and antagonistically in hFTSECs. A first-in-human clinical trial of PRIMA-1^{MET} infusion in cancer patients found that PRIMA-1^{MET} reached its target in tumors and was relatively safe with fully reversible central nervous system-related side effects. The DRI's generated in the present work indicates that these side effects can be reduced or eliminated by combination treatment with SHetA2 allowing lower doses of PRIMA-1^{MET} to be used.

The present work indicates that SHetA2 and PRIMA-1/PRIMA-1^{MET} drug combinations induce apoptotic cell death by pushing cells past the "point of no return" to a ratio of ROS/ATP that is increased beyond a point at which recovery is impossible as defined by the Nomenclature Committee on Cell Death. Both wild type and m-p53 proteins appear to be involved in this mechanism as ovarian cancer cells expressing wild type p53, or endogenous or exogenous m-p53, exhibited greater ROS/ATP ratio elevation upon drug combination treatment compared to cells that were p53 null or had reduced p53. SLC7A11 levels were inversely associated with PRIMA-1 sensitivity, but not SHetA2/PRIMA-1 sensitivity, possibly due to the SHetA2 induction of SLC7A11 expression. In oesophageal cancer, m-p53 increased PRIMA-1^{MET} sensitivity, m-p53/NRF2 complexes repressed SLC7A11 expression and SLC7A11 overexpression decreased, while knockdown increased, PRIMA-1^{MET} sensitivity in m-p53 cells.

Current ovarian cancer maintenance therapy is limited by toxicity and no proven impact on overall survival. To study a maintenance strategy targeted at missense mutant p53, we hypothesized that release of mutant p53 from mortalin inhibition by the SHetA2 drug combined with reactivation of mutant p53 with the PRIMA-1^{MET} drug inhibits growth and tumor establishment synergistically in a mutant-p53 dependent manner. TCGA data and serous ovarian tumors were evaluated for TP53 and HSPA9/mortalin status. SHetA2 and PRIMA-1^{MET} were tested in ovarian cancer cell lines and fallopian tube secretory epithelial cells using isobolograms, fluorescent cytometry, Western blots and ELISAs. Drugs were administered to mice after peritoneal injection of MESOV mutant p53 ovarian cancer cells and prior to tumor establishment, which was evaluated by logistic regression. Fifty-eight percent of TP53 mutations were missense and there were no mortalin mutations in TCGA high-grade serous ovarian cancers. Mortalin levels were sequentially increased in serous benign, borderline and carcinoma tumors. SHetA2 caused p53 nuclear and mitochondrial accumulation in cancer, but not in healthy, cells. Endogenous or exogenous mutant p53 increased SHetA2 resistance. PRIMA-1^{MET} decreased this resistance and interacted synergistically with SHetA2 in mutant and wild type p53-expressing cell lines in association with elevated ROS/ATP ratios. Tumor-free rates in animals were 0% (controls), 25% (PRIMA1^{MET}), 42% (SHetA2) and 67% (combination). SHetA2 (p=0.004) and PRIMA1^{MET} (p=0.048) functioned additively in preventing tumor development with no observed toxicity.

### Example 2: SHetA2 - Palbociclib Drug Combination

CDK 4/6 inhibitors (e.g., Palbociclib, Ribociclib and Abemaciclib) are structurally-related drugs currently FDA approved for treatment of cancer. As a demonstration that SHetA2 works synergistically with the CDK 4/6 group of drugs, SHetA2 was tested for its ability to interact synergistically with palbociclib.

Palbociclib, an FDA approved anticancer drug for treatment of breast cancer, was evaluated in combination with SHetA2 because they inhibit cyclin D1/CDK4/6 complex-driven cancer cell growth through complementary mechanisms. Palbociclib binds and inhibits the CDK4/6 kinase activity. SHetA2 disrupts mortalin complexes and mortalin is known to bind cyclin D1. Another SHetA2 binding target, Hsc70, stabilizes the cyclin D1/CDK4/6 complex. Furthermore, SHetA2 causes degradation of cyclin D1 in cell lines, and in animal models in association with tumor reduction.

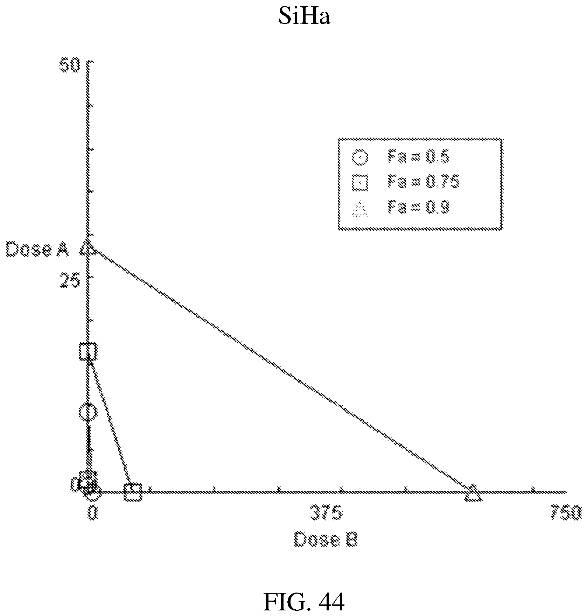

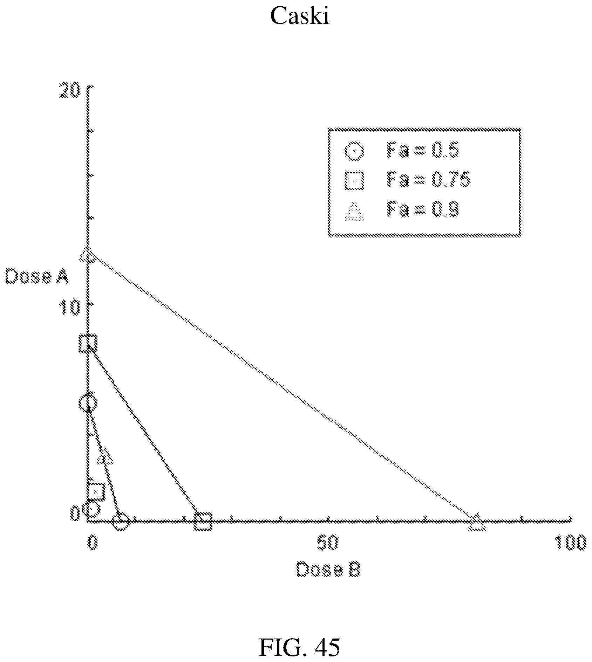

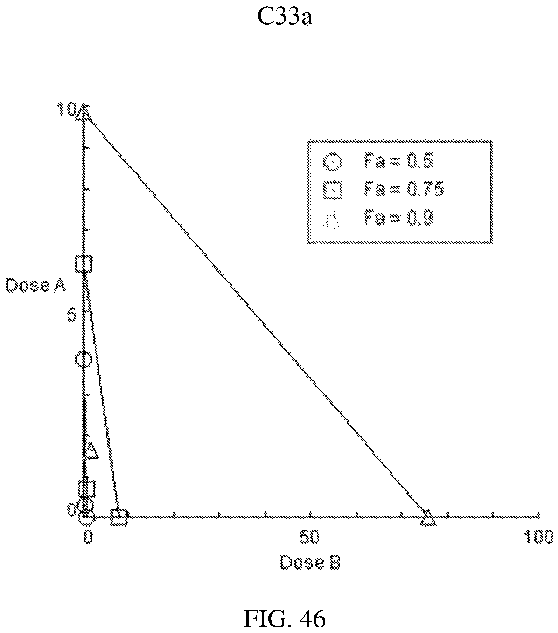

Strong synergy was observed between SHetA2 and Palbociclib when used to treat three human cervical cell lines (SiHa, Caski, C33a) (FIGS. 44-46 and Table 3). Combination Indices (CI's) were derived to determine the level of synergy using the Chow and Talalay method. All of the cell lines exhibited CI's indicative of strong synergy at effective doses (EDs) causing 95% (ED95), 90% (ED90) and 75% (ED75) cell reduction (Tables 3 and 4). The CI's at the ED50 were indicative of strong synergy for the SiHa and Caski cell lines, and synergy for the C33a cell line (Tables 3 and 4). The Dose Reduction Indices (DRIs) were derived to determine the level of reduction for one drug that could be allowed by addition of the second drug at various fold effects (Fa's) of the combination treatment (Table 5).

**Table 3. Combination Index for Various Effective Doses (ED) of SHetA2 and Palbociclib Cervical Cancer Cell Lines**

| Cell Line | ED50 | ED75 | ED90 | ED95 |
|---|---|---|---|---|
| SiHa | 0.205 | 0.113 | 0.093 | 0.089 |
| Caski | 0.218 | 0.238 | 0.296 | 0.361 |
| C33a | 0.512 | 0.222 | 0.195 | 0.221 |

**Table 4. Interpretation of Combination Index (CI)**

| CI Range | Interpretation |
|---|---|
| < 0.1 | Very Strong Synergism |
| 0.1 - 0.3 | Strong Synergism |
| 0.3 - 0.7 | Synergism |
| 0.7 - 0.85 | Moderate Synergism |
| 0.85 - 0.90 | Slight Synergism |
| 0.90 - 1.10 | Nearly Additive |
| 1.10 - 1.20 | Slight Antagonism |
| 1.20 - 1.45 | Moderate Antagonism |
| 1.45 - 3.3 | Antagonism |
| 3.3 - 10 | Strong Antagonism |

**Table 5. Dose Reduction Index (DRI) of SHetA2-Palbociclib Combinations at Various Fold Effects (Fas) in Cervical Cancer**

| Fold Effect (Fa) | SHetA2 Dose | Palbociclib Dose | SHetA2 DRI | Palbociclib DRI |
|---|---|---|---|---|
| 0.05 | 5.44 | 7.16 | 8.76 | 9.6 |
| 0.75 | 8.23 | 24.03 | 5.92 | 14.42 |
| 0.90 | 12.43 | 80.72 | 4.003 | 21.66 |
| 0.95 | 16.47 | 184.00 | 3.068 | 28.57 |

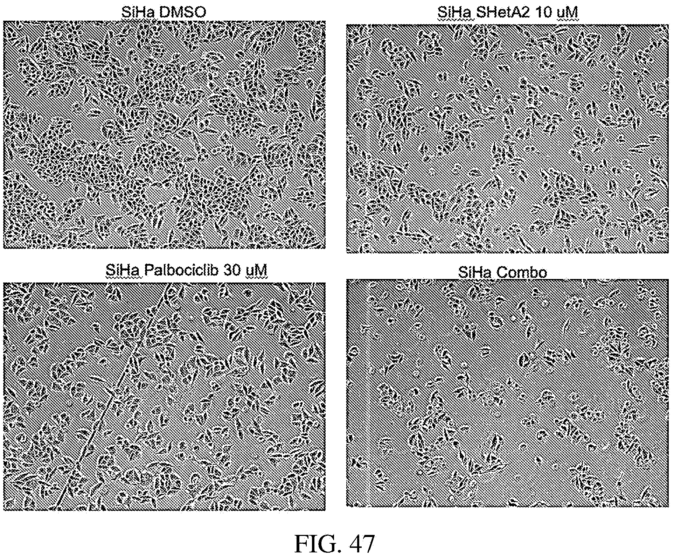

Induction of cell death contributes to the synergistic effect in SiHa and C33A cells (FIGS. 47-48, respectively). An animal model validated the efficacy of SHetA2 and Palbociclib against SiHa human cervical cancer cell line xenografts (FIG. 49). The combination of SHetA2 and palbociclib exerted greater reduction of tumor growth in comparison to palbociclib alone (Repeated Measures ANOVA multiple comparison adjusted p value = 0.001 for comparison of the palbociclib and SHetA2 + palbociclib groups).

SHetA2 is active against all cancers present in the NCI 60-cell line panel and also against cervical and esophageal cancer cell lines. Thus, it is reasonable to expect that the SHetA2 drug combinations with Azabicyclooctan-3-one derivatives, and CDK4/6 inhibitors will also be effective in these cancer types.

## Claims

1. A first compound which is SHetA2, and a second compound selected from PRIMA-1^{met}, PRIMA-1, Palbociclib, Abemaciclib, and Ribociclib, for use in a cancer treatment method comprising conjointly administering to a subject in need of such therapy a therapeutically-effective amount of the first compound and the second compound, wherein the therapeutically-effective amount yields a synergistic effect compared to an effect of the first compound alone and an effect of the second compound alone.

2. A first compound and a second compound for use according to claim 1, wherein the cancer treatment method results in at least one of reduction of tumor size, reduction of tumor growth, inhibition of cancer metastases, cancer cell death, and inhibition of tumor recurrence.

3. A first compound and a second compound for use according to claim 1, wherein the second compound is PRIMA-1^{met} or PRIMA-1.

4. A first compound and a second compound for use according to claim 1, wherein the second compound is Palbociclib, Abemaciclib, or Ribociclib.

5. A first compound and a second compound for use according to claim 1, wherein (i) the first compound is administered in a dose range of 1 mg/kg to 100 mg/kg, and the second compound is administered in a dose range of of 1 mg/kg to 100 mg/kg, or (ii) the first compound and the second compound are administered in a weight-to-weight ratio in a range of 50:1 to 1:50.

6. A synergistic combination comprising a first compound which is SHetA2, and a second compound selected from PRIMA-1^{met}, PRIMA-1, Palbociclib, Abemaciclib, and Ribociclib, wherein the combination provides for a synergistic effect against cancer cells compared to an effect of the first compound alone and an effect of the second compound alone.

7. A kit or commercial package, comprising a first compound which is SHetA2, and a second compound selected from PRIMA-1^{met}, PRIMA-1, Palbociclib, Abemaciclib, and Ribociclib, wherein the first compound and the second compound when administered conjointly provide for a synergistic effect against cancer cells compared to an effect of the first compound alone and an effect of the second compound alone.

8. A synergistic combination according to claim 6, or a kit or commercial package according to claim 7, wherein the inhibition of cancer cells results in at least one of reduction of tumor size, reduction of tumor growth, inhibition of cancer metastases, inhibition of tumor recurrence, cancer cell death.

9. A synergistic combination according to claim 6, or a kit or commercial package according to claim 7, wherein the second compound is PRIMA-1^{met} or PRIMA-1.

10. A synergistic combination according to claim 6, or a kit or commercial package according to claim 7, wherein the second compound is Palbociclib, Abemaciclib, or Ribociclib.

11. A synergistic combination according to claim 6, or a kit or commercial package according to claim 7, wherein (i) the first compound comprises at least one dosage in a range of 1 mg/kg to 100 mg/kg, and the second compound comprises at least one dosage in a range of 1 mg/kg to 100 mg/kg.

12. A synergistic combination according to claim 6, or a kit or commercial package according to claim 7, comprising a dosage of the first compound and the second compound having a weight-to-weight ratio in a range of 50:1 to 1:50.

13. A product comprising a first compound which is SHetA2, and a second compound selected from PRIMA-1^{met}, PRIMA-1, Palbociclib, Abemaciclib, and Ribociclib for simultaneous or sequential use in the treatment of cancer.

## Patentansprüche

1. Erste Verbindung, die SHetA2 ist, und zweite Verbindung, ausgewählt aus PRIMA-1^{met}, PRIMA-1, Palbociclib, Abemaciclib und Ribociclib, für die Verwendung in einem Krebsbehandlungsverfahren, umfassend das gemeinsame Verabreichen einer therapeutisch wirksamen Menge der ersten Verbindung und der zweiten Verbindung an ein Subjekt, das eine solche Therapie benötigt, wobei die therapeutisch wirksame Menge eine synergistische Wirkung im Vergleich zu einer Wirkung der ersten Verbindung allein und einer Wirkung der zweiten Verbindung allein erzeugt.

2. Erste Verbindung und zweite Verbindung für die Verwendung nach Anspruch 1, wobei das Krebsbehandlungsverfahren zu wenigstens einem der Reduktion der Tumorgröße, der Reduktion des Tumorwachstums, der Hemmung von Krebsmetastasen, dem Absterben von Krebszellen und der Hemmung des Wiederauftretens des Tumors führt.

3. Erste Verbindung und zweite Verbindung für die Verwendung nach Anspruch 1, wobei die zweite Verbindung PRIMA-1^{met} oder PRIMA-1 ist.

4. Erste Verbindung und zweite Verbindung für die Verwendung nach Anspruch 1, wobei die zweite Verbindung Palbociclib, Abemaciclib oder Ribociclib ist.

5. Erste Verbindung und zweite Verbindung für die Verwendung nach Anspruch 1, wobei (i) die erste Verbindung in einem Dosisbereich von 1 mg/kg bis 100 mg/kg verabreicht wird und die zweite Verbindung in einem Dosisbereich von 1 mg/kg bis 100 mg/kg verabreicht wird, oder (ii) die erste Verbindung und die zweite Verbindung in einem Gewichtsverhältnis in einem Bereich von 50 : 1 bis 1 : 50 verabreicht werden.

6. Synergistische Kombination, umfassend eine erste Verbindung, die SHetA2 ist, und eine zweite Verbindung, ausgewählt aus PRIMA-1^{met}, PRIMA-1, Palbociclib, Abemaciclib und Ribociclib, wobei die Kombination eine synergistische Wirkung gegen Krebszellen im Vergleich zu einer Wirkung der ersten Verbindung allein und einer Wirkung der zweiten Verbindung allein bereitstellt.

7. Kit oder handelsübliche Packung, umfassend eine erste Verbindung, die SHetA2 ist, und eine zweite Verbindung, ausgewählt aus PRIMA-1^{met}, PRIMA-1, Palbociclib, Abemaciclib und Ribociclib, wobei die erste Verbindung und die zweite Verbindung bei gemeinsamer Verabreichung eine synergistische Wirkung gegen Krebszellen im Vergleich zu einer Wirkung der ersten Verbindung allein und einer Wirkung der zweiten Verbindung allein bereitstellen.

8. Synergistische Kombination nach Anspruch 6 oder Kit oder handelsübliche Packung nach Anspruch 7, wobei die Hemmung von Krebszellen zu wenigstens einem der Reduktion der Tumorgröße, der Reduktion des Tumorwachstums, der Hemmung von Krebsmetastasen, der Hemmung des Wiederauftretens des Tumors, dem Absterben von Krebszellen führt.

9. Synergistische Kombination nach Anspruch 6 oder Kit oder handelsübliche Packung nach Anspruch 7, wobei die zweite Verbindung PRIMA-1^{met} oder PRIMA-1 ist.

10. Synergistische Kombination nach Anspruch 6 oder Kit oder handelsübliche Packung nach Anspruch 7, wobei die zweite Verbindung Palbociclib, Abemaciclib oder Ribociclib ist.

11. Synergistische Kombination nach Anspruch 6 oder Kit oder handelsübliche Packung nach Anspruch 7, wobei (i) die erste Verbindung wenigstens eine Dosierung in einem Bereich von 1 mg/kg bis 100 mg/kg umfasst und die zweite Verbindung wenigstens eine Dosierung in einem Bereich von 1 mg/kg bis 100 mg/kg umfasst.

12. Synergistische Kombination nach Anspruch 6 oder Kit oder handelsübliche Packung nach Anspruch 7, umfassend eine Dosierung der ersten Verbindung und der zweiten Verbindung mit einem Gewichtsverhältnis in einem Bereich von 50 : 1 bis 1 : 50.

13. Produkt, umfassend eine erste Verbindung, die SHetA2 ist, und eine zweite Verbindung, ausgewählt aus PRIMA-1^{met}, PRIMA-1, Palbociclib, Abemaciclib und Ribociclib, für die gleichzeitige oder aufeinanderfolgende Verwendung bei der Behandlung von Krebs.

## Revendications

1. Premier composé qui est SHetA2, et deuxième composé sélectionné parmi PRIMA-1^{met}, PRIMA-1, le palbociclib, l'abémaciclib, et le ribociclib, pour une utilisation dans un procédé de traitement du cancer comprenant l'administration conjointe à un sujet qui nécessite une telle thérapie d'une quantité thérapeutiquement efficace du premier composé et du deuxième composé, dans laquelle la quantité thérapeutiquement efficace donne un effet synergique comparativement à un effet du premier composé seul et un effet du deuxième composé seul.

2. Premier composé et deuxième composé pour une utilisation selon la revendication 1, dans laquelle le procédé de traitement du cancer a pour résultat au moins l'une parmi la réduction de la taille tumorale, la réduction de la croissance tumorale, l'inhibition des métastases cancéreuses, la mort de cellules cancéreuses, et l'inhibition de la récidive tumorale.

3. Premier composé et deuxième composé pour une utilisation selon la revendication 1, dans laquelle le deuxième composé est PRIMA-1^{met} ou PRIMA-1.

4. Premier composé et deuxième composé pour une utilisation selon la revendication 1, dans laquelle le deuxième composé est le palbociclib, l'abémaciclib, ou le ribociclib.

5. Premier composé et deuxième composé pour une utilisation selon la revendication 1, dans laquelle (i) le premier composé est administré dans une plage de dose de 1 mg/kg à 100 mg/kg, et le deuxième composé est administré dans une plage de dose de 1 mg/kg à 100 mg/kg, ou (ii) le premier composé et le deuxième composé sont administrés selon un rapport poids-poids dans une plage de 50:1 à 1:50.

6. Combinaison synergique comprenant un premier composé qui est SHetA2, et un deuxième composé sélectionné parmi PRIMA-1^{met}, PRIMA-1, le palbociclib, l'abémaciclib, et le ribociclib, dans laquelle la combinaison assure un effet synergique contre des cellules cancéreuses comparativement à un effet du premier composé seul et un effet du deuxième composé seul.

7. Kit ou ensemble commercial, comprenant un premier composé qui est SHetA2, et un deuxième composé sélectionné parmi PRIMA-1^{met}, PRIMA-1, le palbociclib, l'abémaciclib, et le ribociclib, dans lequel le premier composé et le deuxième composé, lorsqu'ils sont administrés conjointement, assurent un effet synergique contre des cellules cancéreuses comparativement à un effet du premier composé seul et un effet du deuxième composé seul.

8. Combinaison synergique selon la revendication 6, ou kit ou ensemble commercial selon la revendication 7, dans lequel l'inhibition de cellules cancéreuses a pour résultat au moins l'une parmi la réduction de la taille tumorale, la réduction de la croissance tumorale, l'inhibition des métastases cancéreuses, l'inhibition de la récidive tumorale, la mort de cellules cancéreuses.

9. Combinaison synergique selon la revendication 6, ou kit ou ensemble commercial selon la revendication 7, dans lequel le deuxième composé est PRIMA-1^{met} ou PRIMA-1.

10. Combinaison synergique selon la revendication 6, ou kit ou ensemble commercial selon la revendication 7, dans lequel le deuxième composé est le palbociclib, l'abémaciclib, ou le ribociclib.

11. Combinaison synergique selon la revendication 6, ou kit ou ensemble commercial selon la revendication 7, dans lequel (i) le premier composé comprend au moins une posologie dans une plage de 1 mg/kg à 100 mg/kg, et le deuxième composé comprend au moins une posologie dans une plage de 1 mg/kg à 100 mg/kg.

12. Combinaison synergique selon la revendication 6, ou kit ou ensemble commercial selon la revendication 7, comprenant une posologie du premier composé et du deuxième composé ayant un rapport poids-poids dans une plage de 50:1 à 1:50.

13. Produit comprenant un premier composé qui est SHetA2, et un deuxième composé sélectionné parmi PRIMA-1^{met}, PRIMA-1, le palbociclib, l'abémaciclib, et le ribociclib, pour une utilisation simultanée ou séquentielle dans le traitement du cancer.
